# EUROPEAN PATENT SPECIFICATION

(11) **EP 3 520 785 B1**
(45) Date of publication and mention of the grant of the patent: **17.07.2024**
(21) Application number: 17857859.7
(22) Date of filing: 08.10.2017
(51) Int. Cl.: A61K 31/192, A61K 31/19, A61P 15/02, A61K 9/06, A61K 9/08, A61K 45/06, A61P 31/04, A61P 31/10

(54) **USES OF BACTERIOSTATIC AGENT FORMULA IN PREPARING COMPOSITION FOR VAGINAL USE AND COMPOSITION FOR VAGINAL USE**
VERWENDUNGEN EINER BAKTERIOSTATISCHEN WIRKSTOFFFORMEL ZUR HERSTELLUNG EINER ZUSAMMENSETZUNG ZUR VAGINALEN VERWENDUNG UND ZUSAMMENSETZUNG ZUR VAGINALEN VERWENDUNG
UTILISATION D'UNE FORMULE RENFERMANT UN AGENT BACTÉRIOSTATIQUE POUR LA PRÉPARATION D'UN COMPOSÉ À APPLICATION VAGINALE ET LEDIT COMPOSÉ

(30) Priority: 09.10.2016 CN 201610899657
(43) Date of publication of application: 07.08.2019
(73) Proprietor: Shenzhen Eulikan Biotechnology Co., Ltd., Shenzhen, Guangdong 51800 (CN); Singapore ZE&Z International Pte. Ltd., Singapore City 079903 (SG)
(72) Inventor: ZENG, Zhongming, Singapore City 079903 (CN); ZHANG, Wenyong, Shenzhen Guangdong 518000 (CN); WU, Kongyuan, Shenzhen Guangdong 518000 (CN)
(74) Representative: Zenz Patentanwälte Partnerschaft mbB
(86) International application number: PCT/CN2017/105296
(87) International publication number: WO 2018/064978

(56) References cited:
- WO-A1-2011/041938
- WO-A1-2013/121449
- WO-A1-2014/100851
- CN-A- 1 215 330
- CN-A- 1 688 329
- CN-A- 101 437 504
- CN-A- 101 744 833
- CN-A- 104 548 071
- HUGBO P G: "Additive and synergistic actions of equipotent admixtures of some antimicrobial agents", PHARMACEUTICA ACTA HELVETIAE, vol. 51, no. 10, 1 February 1976 (1976-02-01), pages 184 - 288, XP009519830

## Description

### TECHNICAL FIELD

The present disclosure relates to a use of an antimicrobial agent combination in preparing vaginal composition. The combination of antimicrobial agents can be used for preparing various therapeutic or non-therapeutic compositions for vagina. The present disclosure also relates to a composition for vagina, which can be used for vaginal health care, and prevention and treatment of vaginal infection.

### BACKGROUND ART

Gram-positive bacilli referred to as "normal vaginal flora", dominate on vaginal mucosa surface in the healthy woman. Such dominating Gram-positive bacilli, the main type of which is lactobacillus, can metabolize glycogen in vaginal mucosa epithelial cells to produce acid, thereby keep the pH value of the vaginal in a range from 3.5 to 4.3, which is essential for the female genital tract to resist bacterial infections.

The vaginal flora may be disturbed by various factors so to decrease the vagina's resistance to infections. As a result, vaginal infections are very common, such as bacterial vaginosis, aerobic vaginitis, and Candidal vaginitis, etc.. These infectious disorders frequently result from the increase of bacteria such as Escherichia coli, Staphylococcus aureus, group B Streptococcus, Gardnerella vaginalis, Prevotella, and Candida. Therefore it is of great clinical significance to genital tract infection prevention and treatment to avoid inhibiting or destroying beneficial lactobacilli in the course of antimicrobial treatment against pathogenic bacteria and fungi.

However currently, there is still lack of effective technology or method which may not only selectively inhibit bacteria such as Escherichia coli and Staphylococcus aureus, but also protect and promote beneficial lactobacilli in the vagina.

### SUMMARY

The object of the present disclosure is to provide an antimicrobial agent composition that has a strong inhibition effect on Escherichia coli, and Staphylococcus aureus. When being used in the vagina, the composition may promote the restoration of beneficial Lactobacilli and increase vaginal acidity.

The development of technologies for selectively inhibiting pathogenic bacteria while promoting beneficial lactobacilli remains a frontier topic in the field of gynecologic infections. According to the researches by the inventor, any one of 2-phenylethanol, benzoic acid and/or its sodium salt, and propionic acid and/or its salt could selectively inhibit the growth of Lactococcus lactis while having no significant inhibition effect against Lactobacillus. The inhibition effect of other antimicrobial agents such as acetic acid, dehydroacetic acid, and caprylic acid separately against Lactococcus lactis as well as Lactobacillus, is not selective.

It has been found out by the research that 2-phenylethanol, combined with benzoic acid and/or its sodium salt, or 2-phenylethanol, combined with propionic acid and/or its salt, exhibits a synergistic inhibition effect against Escherichia coli and/or Staphylococcus aureus. Further, it gets stronger the inhibiting action against Escherichia coli and/or Staphylococcus aureus when the following three types of agents are combined: (1) 2-phenylethanol; (2) benzoic acid and/or its sodium salt; (3) propionic acid and/or its salt.

However, the combination of 2-phenylethanol with some other antimicrobial agents such as dehydroacetic acid and/or its sodium salt, or sorbic acid and/or its potassium salt, is less effective than the combination as mentioned above.

The present disclosure provides a composition for vagina for use in promoting the restoration of beneficial Lactobacilli, increasing vaginal acidity, eliminating and/or alleviating vaginal discomforts comprising vulvovaginal pruritus or pain, and/or sexual intercourse pain, improving leucorrhea properties, eliminating leucorrhea smell, and/or preventing and/or treating vaginal infectious diseases comprising cytolytic vaginosis, vaginal dysbacteriosis, atrophic vaginitis, aerobic vaginitis, and/or bacterial vaginosis, the composition includes the following antimicrobial agents: (1) 2-phenylethanol, the total content of which is 0.05 to 0.60 percent(weight/volume); (2) propionic acid and/or sodium propionate and/or calcium propionate, the total content of which, calculated as sodium propionate, is 0.25 to 0.70 percent(weight/volume), and/or benzoic acid and/or sodium benzoate, the total content of which, calculated as sodium benzoate, is 0.05 percent to 0.15 percent(weight/volume); wherein the pH value of the composition is from 3.0 to 4.3, and the dosage form of the composition is a solution, a water-soluble gel, or an emulsive ointment.

The propionic acid and it's homologous according to the present disclosure belong to the short-chain fatty acid preservatives. The preferred is propionic acid and/or its sodium salt.

According to the present disclosure, the content of benzoic acid, calculated as sodium benzoate refers to that the content of benzoic acid, etc. is converted to that of sodium benzoate. The method is to multiply the content of benzoic acid, etc. with the molecular weight ratio of sodium benzoate to benzoic acid, etc., and the result obtained is the content of sodium benzoate converted from the content of benzoic acid, etc. For example, the molecular weight of sodium benzoate is 144. The molecular weight of benzoic acid is 122. Then 0.1 percent (weight/volume) of benzoic acid , is converted into 0.12 percent (weight/volume) of sodium benzoate. And so on and vice verse.

According to the present disclosure, the content of propionic acid and/or its calcium salt calculated as sodium propionate refers to that the content of propionic acid, etc. is converted to that of sodium propionate. The method is to multiply the content of propionic acid, etc. with the molecular ratio of sodium propionate to propionic acid, etc., and the result obtained is the content of sodium propionate converted from the content of propionic acid, etc. For example, the molecular weight of sodium propionate is 96, the molecular weight of propionic acid is 74. Then 0.31 percent (weight/volume) of propionic acidis converted into 0.4 percent (weight/volume) of sodium propionate. And so on and vice versa.

Preferably, the total content of 2-phenylethanol is 0.10 to 0.40 percent (weight/volume); the total content of propionic acid and/or sodium propionate and/or calcium propionate, calculated as sodium propionate, is 0.525 to 0.575 percent (weight/volume); the total content of benzoic acid and/or sodium benzoate, calculated as sodium benzoate, is 0.065 to 0.090 percent (weight/volume).

Preferably, the composition includes (1) 2-phenylethanol; (2) propionic acid and/or sodium propionate and/or calcium propionate; (3) benzoic acid and/or sodium benzoate .

Preferably, the antimicrobial agents are 2-phenylethanol, propionic acid and/or sodium propionate, and benzoic acid and/or sodium benzoate.

Preferably, the composition further includes one or more antimicrobial agents selected from a group consisting of dehydroacetic acid, sodium dehydroacetate, sorbic acid, potassium sorbate, sodium sorbate, diacetic acid, sodium diacetate, caprylic acid, sodium caprylate, capric acid, sodium caprate, undecylenic acid, sodium undecylenate, lauric acid, sodium laurate, natamycin, lactoferrin, lactoferrin peptide, lysozyme, antibacterial protein, antibacterial peptide, lichenic acid, bergenin, tropolone, chlorogenic acid, palmatine, benzyl alcohol, propylene phenoxyethanol, 1,2 - pentanediol, 1,2 - hexanediol, 1,6 - hexanediol, 1,2 - octanediol, 1,2 - decanediol, 2-methyl-1,3-propanediol, ethylhexylglycerin, and benzoylperoxide. The antimicrobial agents are used to further enhance the antimicrobial effect of the vaginal composition of the present disclosure against Staphylococcus aureus, Escherichia coli, as well as against other pathogenic microorganisms, such as virus HIV, HPV, etc.

Preferably, the composition further includes one or more saccharides selected from the following group consisting of glucose, fructose, mannose, sucrose, isomaltulose, 1-kestose, nistose trihydrate, 1F-fructofuranosylnystose, maltose, isomaltose, isomaltotriose, isomaltotetraose, isomaltopentaose, trehalose, cellobiose, melibiose, gentiobiose, gentiooligsaccharide, raffinose, panose, maltooligosaccharide, isomaltooligsaccharide, fructooligosaccharide, glucomannan, dextrin, starch, and glycogen, with a total content of 1.0 to 9.0 percent (weight/volume). The saccharide-contained vaginal composition of the present disclosure can modulate vaginal flora biphasically, that is, it can selectively inhibit Escherichia coli and/or Staphylococcus aureus etc. It can also selectively promote beneficial Lactobacilli in the vagina. Compared with the prior art, the vaginal composition of the present disclosure has the advantages of modulating vaginal flora, enhancing the vaginal acidity, and maintaining vaginal beneficial Lactobacilli. Thus it can enhance the vaginal resistance to infections and can be used for prevention and/or treatment of vaginal Lactobacilli reduction, or vaginal dysbacteriosis, or bacterial vaginosis, or for restoring normal vaginal flora after antibacterial treatment of genital tract infection, or as an adjuvant treatment of genital tract infection. It has also been found out by the inventor that when the total content of saccharides is less than 1 percent (weight/volume), the composition is weak in promoting Lactobacilli growth and acid production; and when the total content of saccharides is higher than 9 percent (weight/volume), it may be irritative to vaginal mucosa.

Preferably, the composition for vagina further includes one or more amino acids selected from a group consisting of glutamic acid, glutamine, aspartic acid, asparagine, isoleucine, phenylalanine, valine, leucine, proline, and threonine, with a total content of 0.1 to 10.0 percent (weight/volume). The amino acids contained composition for vagina of the present disclosure can reduce acid production by vaginal flora, and can be used to treat or assist in the treatment of cytolytic vaginosis. It is also found out by the inventor that when the total content of amino acid is less than 0.1 percent (weight/volume), the composition is weak in decreasing acid production by vaginal flora; and when the total content of amino acids is higher than 10 percent (weight/volume), it may cause abnormal change of vaginal flora.

Preferably, the composition further includes one or more estrogen and/or phytoestrogen agents selected from a group consisting of diethylstilbestrol, estradiol, estriol, daidzein, aglycone of daidzein, genistin, genistein, glycitein, aglycone of glycitein, biochanin, coumestrol, and formononetin, with a total content of 0.001 to 1.0 percent (weight/volume). The vaginal composition of the present disclosure that contains estrogen and/or phytoestrogen can promote the vaginal mucosa epithelial cells to synthesize glycogen, maintain or restore the normal vaginal microenvironment, and promote the growth of beneficial lactobacillus to restore the balance of micro-ecosystem.

Preferably, the composition further includes one or more substances selected from a group consisting of 0.01 to 5.0 percent (weight/volume) of aloe extract, 0.01 to 5.0 percent (weight/volume) of lavender extract, 0.001 to 1.0 percent (weight/volume) of vitamin E, 0.001 to 1.0 percent (weight/volume) of vitamin A, 0.001 to 1.0 percent (weight/volume) of vitamin D, and 0.001 to 1.0 percent (weight/volume) of vitamin C. The composition for vagina prepared by the present disclosure that includes the above substances may be used for protecting vaginal mucosa and promoting the repair of damaged vaginal mucosa.

Preferably, the dosage form of the composition is a solution, a water-soluble gel, or an emulsive ointment, in which the pH value of the composition is within a range from 3.0 to 4.3.

According to the use of the present disclosure, the composition for vagina use may be a non-therapeutic vaginal health product, a vaginal use commodity, a vaginal health care product, a vaginal nursing product, a vaginal cosmetic, a vaginal sanitary product, or a vaginal cleaning product; or the composition for vagina use may be a therapeutic vaginal health product, a vaginal medical device, a vaginal disinfection device, a vaginal drug-device, or a vaginal drug; or the composition for vagina may be a lotion, a detergent, a curing agent, an odor removing agent, or an antipruritic agent, a refreshing agent, a wetting agent, a lubricant, a disinfectant, a fungicide, an antimicrobial agent, a mucosal surface microbicide, a microecological modulator, or a microbial modulator, for vagina.

The present disclosure also provides a composition for vagina, the composition includes: (1) 2-phenylethanol, the total content of which is from 0.05 to 0.6 percent (weight/volume); (2) propionic acid and/or sodium propionate and/or calcium propionate, the total content of which, calculated as sodium propionate, is from 0.25 to 0.70 percent (weight/volume), and/or benzoic acid and/or sodium benzoate, the total content of which, calculated as sodium benzoate, is 0.05 to 0.15 percent (weight/volume).

The dosage form of the composition is a solution, a water-soluble gel, or an emulsive ointment, in which the pH value of the composition is within a range from 3.0 to 4.3.

Preferably, a total content of 2-phenylethanol is from 0.10 to 0.40 percent (weight/volume); a total content of propionic acid and/or sodium propionate and/or calcium propionate, calculated as sodium propionate, is from 0.525 to 0.575 percent (weight/volume); and/or a total content of benzoic acid and/or sodium benzoate, calculated as sodium benzoate, is from 0.065 to 0.090 percent (weight/volume).

Preferably, the composition includes: (1) 2-phenylethano; (2) propionic acid and/or sodium propionate and/or calcium propionate; (3) benzoic acid and/or sodium benzoate.

Preferably, the antimicrobial agents are 2-phenylethanol, propionic acid and/or sodium propionate, and benzoic acid and/or sodium benzoate.

Preferably, the composition further includes one or more antimicrobial agents selected from a group consisting of dehydroacetic acid, sodium dehydroacetate, sorbic acid, potassium sorbate, sodium sorbate, diacetic acid, sodium diacetate, caprylic acid, sodium caprylate, capric acid, sodium caprate, undecylenic acid, sodium undecylenate, lauric acid, sodium laurate, natamycin, lactoferrin, lactoferrin peptide, lysozyme, antibacterial protein, antibacterial peptide, lichenic acid, bergenin, tropolone, chlorogenic acid, palmatine, benzyl alcohol, propylene phenoxyethanol, 1,2-pentanediol, 1,2-hexanediol, 1,6-hexanediol, 1,2-octanediol, 1,2-decanediol, 2-methyl-1,3-propanediol, ethylhexylglycerin, and benzoyl peroxide.

Preferably, the composition also includes one or more estrogen and/or phytoestrogen agents selected from a group consisting of diethylstilbestrol, estradiol, estriol, daidzein, aglycone of daidzein, genistin, genistein, glycitein, aglycone of glycitein, biochanin, coumestrol, and formononetin, with a total content of 0.001 to 1.0 percent (weight/volume).

Preferably, the composition also includes one or more saccharides selected from a group consisting of glucose, fructose, mannose, sucrose, isomaltulose, 1-kestose, nistose trihydrate, 1F-fructofuranosylnystose, maltose, isomaltose, isomaltotriose, isomaltotetraose, isomaltopentaose, trehalose, cellobiose, melibiose, gentiobiose, gentiooligosaccharide, raffinose, panose, maltooligosaccharide, isomaltooligosaccharide, fructooligosaccharide, glucomannan, dextrin, starch, and glycogen, with a total content of 1.0 to 9.0 percent (weight/volume).

Preferably, the saccharide is one or more saccharides selected from the group consisting of glucose, fructose, mannose, sucrose, maltose, isomaltulose, isomaltose, trehalose, and maltooligosaccharide, with a total content of 2.0 to 6.5 percent (weight/volume).

Preferably, the composition further includes one or more substances selected from a group consisting of 0.01 to 5.0 percent (weight/volume) of aloe extract, 0.01 to 5.0 percent (weight/volume) of lavender extract, 0.001 to 1.0 percent (weight/volume) of vitamin E, 0.001 to 1.0 percent (weight/volume) of vitamin A, 0.001 to 1.0 percent (weight/volume) of vitamin D, and 0.001 to 1.0 percent (weight/volume) of vitamin C.

Preferably, the composition further includes one or more amino acids and/or physiologically acceptable salts thereof, which is selected from a group consisting of glutamic acid, glutamine, aspartic acid, asparagine, isoleucine, phenylalanine, valine, leucine, proline, and threonine, with a content of 0.1 to 10.0 percent (weight/volume).

Preferably, the total content of amino acid is from 1.0 to 5.0 percent (weight/volume), and the amino acid is glutamic acid and/or aspartic acid.

The composition of the present disclosure may be in various packages, including but not limited to a sealed and sterilized single dose package or the composition is packaged in a vaginal device or a disposable vaginal applicator. Sealed and sterilized single dose package is preferable. The producing processes, method, and the choice of adjuvant are all known to those skilled in the art based on the disclosure of the present disclosure.

The composition of the present disclosure may optionally comprise a low-dose antimicrobial drug such as metronidazole or tinidazole, etc. The content of metronidazole or tinidazole in single unit dosage or single unit package or unit dosage form such as a tablet, a suppository, or a single dosage ointment is from 0.001 to 0.5 milligrams, preferably 0.01 to 0.25 milligrams. The composition of the present disclosure including low dosage metronidazole or tinidazole has the effects of nursing and/or modulating vaginal micro-ecosystem, inhibiting anaerobic bacteria, and keeping vaginal micro-ecosystem in balance.

The present disclosure also provides a method for manufacturing a vaginal composition, which includes the following steps: adding the following agents into water, and/or water-soluble gel matrix, and/or emulsive ointment matrix: (1) 2-phenylethanol, the total content of which is 0.05 to 0.6 percent (weight/volume); (2) propionic acid and/or sodium propionate and/or calcium propionate, the total content of which, calculated as sodium propionate, is 0.25 to 0.7 percent (weight/volume), and/or benzoic acid and/or sodium benzoate, the total content of which, calculated as sodium benzoate, is 0.05 to 0.15 percent (weight/volume); wherein the pH value of the composition is from 3.0 to 4.3, and the dosage form of the composition is a solution, a water-soluble gel, or an emulsive ointment.

For example, viscous, non-flowable, water-soluble gel matrixes can be used in the preparation of the gel composition, such as Carbomer, and/or methylcellulose (MC), and/or glucomannan, and/or tragacanth gum, and/or west tragacanth gum, and/or Xanthan gum, among which Xanthan gum is preferred. The gel composition can be manufactured according to a method known to those skilled in the art. For example, the following agents will be weighed proportionally according to the technological process: (1) 2-phenylethanol; (2) propionic acid, and/or sodium propionate and/or calcium propionate; and/or benzoic acid and/or sodium benzoate; (3) other components, and (4) Xanthan gum. Homogeneously mixing the above components. Quantitatively adding distilled water and stirring to dissolve the components and to swell Xanthan gum so to form a homogenous gel. Adjusting the pH value of the composition to a range from 3.0 to 4.3 with acid and/or alkali. Further sterilization can be carried out. There are a variety of sterilization processes to choose from: radiation sterilization, intermittent sterilization (such as sterilization at 60°C for 15 minutes, then at 36 °C for 6 to 12 hours, then at 60°C for 15 minutes, then at 36°C for 6 to 12hours, and finally at 60 °C for 15 minutes). Or the components such as 2-phenylethanol, sodium propionate, and sodium benzoate are separately prepared into solution, filtered and sterilized, and then added to the sterilized water-soluble colloidal matrix.

For example, when preparing an emulsive ointment, a matrix substance known to those skilled in the art can be chosen, such as glyceryl monostearate, and/or paraffin wax, and/or beeswax, and/or white vaseline, and/or sodium lauryl sulfate, and/or polysorbate, etc.

For example, when preparing a solution, the above-mentioned components other than a water-soluble colloidal matrix or an emulsive ointment matrix can be mixed, watered, dissolved, sterilized, and reserved. Cotton balls or tampons can be soaked with the solution and used vaginally. Or can be made into a vaginal lotion.

According to the technical proposals of the present disclosure, the composition of the present disclosure can be used to eliminate or alleviate vaginal discomfort such as vulvovaginal pruritus, pain, and/or sexual intercourse pain, and/or to improve leucorrhea properties, and/or eliminate leucorrhea smell, and/or clean and care vagina.

Accordingly, the present disclosure also relates to a composition for vagina for use in preventing and/or treating vaginal infectious diseases. The vaginal infectious diseases include, but are not limited to, cytolytic vaginosis, vaginal dysbacteriosis, atrophic vaginitis, aerobic vaginitis, and bacterial vaginosis. The composition is adapted to be applied to the vagina of a woman in need. For example, the composition of the water-soluble gel dosage form of the present disclosure is adapted to be applied into the vagina with an injector, or the composition of solution dosage form is adapted to be soaked by a vaginal tampon or a cotton ball or a vaginal plug, and then placed into the vagina.

### DETAILED DESCRIPTION

### Composition Examples

### Example 1:

The water-soluble gel composition of the present disclosure can be prepared by the following method: homogeneously mixing 0.05grams sodium benzoate, 0.25grams sodium propionate, and 1.0gram Xanthan gum, adding 100 milliliters distilled water and stirring to dissolve sodium benzoate and sodium propionate and to swell the Xanthan gum into a uniform viscous gel. Then adding 0.05 grams 2-phenylethanol, adjusting the pH value of the solution to 3.0, and finally heating at 60°C for 15 minutes.

### Example 2:

The 100 milliliters composition can be prepared basically according to the technological processes of Example 1 by weighing the ingredients according to the following ratio:

| | |
|---|---|
| 2-Phenylethanol | 0.60 percent (weight/volume) |
| Sodium propionate | 0.70 percent (weight/volume) |
| Glucose | 1.00 percent (weight/volume) |
| Xanthan gum | 2.15 percent (weight/volume) |
| Distilled water | 100 milliliters |
| pH | 4.0 |

### Example 3:

The 100 milliliters composition can be prepared basically according to the technological processes of Example 1 by weighing the ingredients according to the following ratio:

| | |
|---|---|
| 2-Phenylethanol | 0.06 percent (weight/volume) |
| Sodium propionate | 0.55 percent (weight/volume) |
| Sodium benzoate | 0.12 percent (weight/volume) |
| Maltose | 6.50 percent (weight/volume) |
| Xanthan gum | 2.0 percent (weight/volume) |
| Distilled water | 100 milliliters |
| pH | 3.2 |

### Example 4:

The 100 milliliters composition can be prepared basically according to the technological processes of Example 1 by weighing the ingredients according to the following ratio:

| | |
|---|---|
| 2-Phenylethanol | 0.50 percent (weight/volume) |
| Sodium benzoate | 0.075 percent (weight/volume) |
| Distilled water | 100 milliliters |
| pH | 3.5 |

### Example 5:

The 100 milliliters composition can be prepared basically according to the technological processes of Example 1 by weighing the ingredients according to the following ratio:

| | |
|---|---|
| 2-phenylethanol | 0.45 percent (weight/volume) |
| Sodium propionate | 0.60 percent (weight/volume) |
| Distilled water | 100 milliliters |
| pH | 4.0 |

### Example 6:

The 100 milliliters composition can be prepared basically according to the technological processes of Example 1 by weighing the ingredients according to the following ratio:

| | |
|---|---|
| 2-Phenylethanol | 0.35 percent (weight/volume) |
| Sodium propionate | 0.55 percent (weight/volume) |
| Glutamic acid | 3.0 percent |
| Aspartic acid | 3.0 percent |
| Xanthan gum | 1.5 percent(weight/volume) |
| Distilled water | 100 milliliters |
| pH | 4.0 |

### Example 7:

The 100 milliliters composition can be prepared basically according to the technological processes of Example 1 by weighing the ingredients according to the following ratio:

| | |
|---|---|
| 2-Phenylethanol | 0.20 percent (weight/volume) |
| Sodium propionate | 0.575 percent (weight/volume) |
| Sodium benzoate | 0.075 percent (weight/volume) |
| Maltose | 5.00 percent (weight/volume) |
| Xanthan gum | 1.00 percent (weight/volume) |
| Distilled water | 100 milliliters |
| pH | 3.5 |

### Example 8:

The 100 milliliters composition can be prepared basically according to the technological processes of Example 1 by weighing the ingredients according to the following substances:
2-Phenylethanol 0.3 grams, sodium propionate 0.5 grams, sodium benzoate 0.06 grams, and lactoferrin 0.5 grams;
Glutamic acid 0.5 grams, glutamine 0.25 grams, aspartic acid 0.5 grams, asparagine 0.25 grams, isoleucine 0.25 grams, methionine 0.25 grams, phenylalanine 0.25 grams, valine 0.25 grams, leucine 0.25 grams, and proline 0.25 grams;
Xanthan gum 1.5 grams, distilled water 100 milliliters, the pH was adjusted to 3.5.

### Example 9:

The 100 milliliters composition can be prepared basically according to the technological processes of Example 1 by weighing the ingredients according to the following ratio:

| | |
|---|---|
| Phenoxyethanol | 0.05 percent (weight/volume) |
| Sodium propionate | 0.50 percent (weight/volume) |
| Sodium benzoate | 0.15 percent (weight/volume) |
| Xanthan gum | 1.2 percent (weight/volume) |
| Distilled water | 100 milliliters |
| pH | 4.3 |

### Example 10:

The following solution composition can be prepared basically according to the conventional technological processes in the field:

| | |
|---|---|
| 2-Phenylethanol | 0.60 percent (weight/volume) |
| Sodium benzoate | 0.15percent (weight/volume) |
| Sodium propionate | 0.70percent (weight/volume) |
| Distilled water | 100 milliliters |
| pH | 4.0 |

### Experimental Example

### Experimental Example 1

1. Experimental objective: to observe the inhibition effects of each of the following agents in a specific concentration range against Lactococcus lactis and Lactobacillus: 2-phenylethanol, propionic acid and/or its salt, benzoic acid and/or its sodium salt, acetic acid and/or its salt, dehydroacetic acid and/or its salt, and caprylic acid and/or its salt.
2. Experimental method:
2.1 Experimental strains: one Lactococcus lactis strain and one Lactobacillus jensenii strain, both were clinical isolates.
2.2 Preparation of base gel: 1.0 percent Xanthan gum, 1.5 percent isomaltulose, and 10 percent calf serum were added into MRS liquid medium. The pH value was adjusted to 5.0.
2.3 preparing based on the base gel the test gels containing different concentrations of antimicrobial agents, as shown in Tables 1 to 6, then dividing the test gels into tubes, 5ml per tube and reserved.
2.4 Experimental procedure: the test gel tubes containing different concentrations of antimicrobial agents, as shown in Tables 1 to 6 were inoculated with 100 microliters of Lactobacillus lactis suspension and 100 microliters of Lactobacillus jensenii suspension at the same time. The turbidities of both suspensions were 0.5 MCF. Then the tubes were cultivated at 37°C and under 5 percent CO₂ for 32 hours. Then the changes of the pH values of gels were tested. The gels were smeared, Gram stained, and microscopically examined for the morphology and quantity of bacteria.

3. Experimental result:
3.1 As shown in table 1, the 0.35 to 0.45 percent of 2-phenylethanol had a stronger inhibition effect on the growth of Lactococcus lactis and a weaker inhibition effect on the growth of Lactobacillus. However, the pH values were in the range from 4.1 to 4.6 and did not decrease to 3.8, which indicated that acid production was inhibited.

**Table 1. Inhibitory effect of 2-phenylethanol on the growth of Lactococcus lactis and Lactobacillus jensenii**

| Concentration (%) | 0.15 | 0.2 | 0.25 | 0.3 | 0.35 | 0.4 | 0.45 | 0.5 |
|---|---|---|---|---|---|---|---|---|
| pH value | <3.8 | <3.8 | <3.8 | <3.8 | 3.8 -4.1 | 4.4 | 4.6 | 4.8 |
| Smear | G^{+c}++++ | G^{+c}++++ | G^{+c}++++ | G^{+c}++++ | G^{+c}+~++ | G^{+c}+ | G^{+c}0~+ | G^{+c}0~+ |
| | G^{+b}+ | G^{+b}+ | G^{+b}+ | G^{+b}+ | G^{+b}+++ | G^{+b}+++ | G^{+b}++ | G^{+b}+ |

3.2 As shown in table 2, the 0.4 to 0.6 percent of sodium propionate had a stronger inhibition effect on the growth of Lactococcus lactis and a weaker inhibition effect on the growth of Lactobacillus, which indicated that the antimicrobial action was selective. However, the pH value was in the range from 4.4 to 4.6, did not decrease to 3.8, which indicated that acid production was inhibited.

**Table 2. Inhibitory effect of sodium propionate on the growth of Lactococcus lactis and Lactobacillus jensenii**

| Concentration (%) | 0.1 | 0.2 | 0.3 | 0.4 | 0.5 | 0.6 | 0.7 |
|---|---|---|---|---|---|---|---|
| pH value | 3.8 | 3.8 | 3.8 | 4.4 | 4.4 -4.6 | 4.6 | 4.8 |
| | G^{+c}++++ | G^{+c}++++ | G^{+c}++++ | G^{+c}+ | G^{+c}+ | G^{+c}0~+ | G^{+c}0~+ |
| Smear | G^{+b}+ | G^{+b}+ | G^{+b}+ | G^{+b}+++ | G^{+b}+++ | G^{+b}++ | G^{+b}+ |

3.3 As shown in table 3, the 0.065 to 0.1 percent of sodium benzoate had a stronger inhibition effect on the growth of Lactococcus lactis and a weaker inhibition effect on the growth of Lactobacillus, which indicated that the antimicrobial effect was selective. However, the pH values were in the range of 4.1 to 4.6 and did not decrease to 3.8, which indicated that acid production was inhibited.

**Table 3. Inhibitory effect of sodium benzoate on the growth of Lactococcus lactis and Lactobacillus jensenii**

| Concentration (%) | 0.035 | 0.05 | 0.065 | 0.10 |
|---|---|---|---|---|
| pH value | <3.8 | <3.8 | 4.1 | 4.6 |
| Smear | G^{+c}++++ | G^{+c}++++ | G^{+c}++ | G^{+c}+ |
| | G^{+b}+ | G^{+b}+ | G^{+b}++ | G^{+b}+++ |

3.4 As shown in table 4, 0.1 to 0.8 percent of sodium acetate had little inhibition effect on the growth of Lactococcus lactis. Lactococcus lactis grew more, while Lactobacillus grew less.

**Table 4. Inhibitory effect of sodium acetate on the growth of lactococcus lactis and Llactobacillus jensenii**

| Concentration (%) | 0.1 | 0.2 | 0.3 | 0.4 | 0.5 | 0.6 | 0.7 | 0.8 |
|---|---|---|---|---|---|---|---|---|
| pH value | <3.8 | <3.8 | <3.8 | <3.8 | <3.8 | 3.8 | 3.8 | 3.8-4.1 |
| Smear | G^{+c}++++ | G^{+c}++++ | G^{+c}++++ | G^{+c}++++ | G^{+c}++++ | G^{+c}++++ | G^{+c}++++ | G^{+c}++++ |
| | G^{+b}+ | G^{+b}+ | G^{+b}+ | G^{+b}+ | G^{+b}+ | G^{+b}+ | G^{+b}+ | G^{+b}+ |

3.5 As shown in table 5, 0.005 to 0.035 percent of sodium dehydroacetate had little inhibition effect on the growth of Lactococcus lactis. Lactococcus lactis grew more, while Lactobacillus grew less.

**Table 5. Inhibitory effect of sodium dehydroacetate on growth of Lactococcus lactis and Lactobacillus j ensenii**

| Concentration (%) | 0.005 | 0.010 | 0.015 | 0.020 | 0.025 | 0.030 | 0.035 |
|---|---|---|---|---|---|---|---|
| pH value | 3.8 | 3.8 | 3.8 | 3.8 | 3.8 | 3.8 | 3.8 |
| Smear | G^{+c}++++ | G^{+c}++++ | G^{+c}++++ | G^{+c}++++ | G^{+c}++++ | G^{+c}++++ | G^{+c}++++ |
| | G^{+b}+ | G^{+b}+ | G^{+b}+ | G^{+b}+ | G^{+b}+ | G^{+b}+ | G^{+b}+ |

3.6 As shown in table 6, 0.010 to 0.020 percent of sodium caprylate had little inhibition effect on the growth of Lactococcus lactis. Lactococcus lactis grew more, while lactobacillus grew less. However, 0.05 percent of sodium caprylate had a stronger inhibition effect on both of the Lactococcus lactis and lactobacillus.

**Table 6. Inhibitory effects of sodium octanoate on the growth of Lactococcus lactis and Lactobacillus jensenii**

| Concentration (%) | 0.000 | 0.010 | 0.015 | 0.020 | 0.050 |
|---|---|---|---|---|---|
| pH value | <3.8 | <3.8 | <3.8 | 3.8 | 4.8 |
| Smear | G^{+c}++++ | G^{+c}++++ | G^{+c}++++ | G^{+c}++++ | G^{+c}0~+ |
| | G^{+b}+ | G^{+b}+ | G^{+b}+ | G^{+b}+ | G^{+b0}~+ |

4. Conclusion: each one of 2-phenylethanol, propionic acid and/or its salt, and benzoic acid and/or its sodium salt in a specific concentration range had a strong inhibition effect on Lactococcus lactis and a weak inhibition effect on Lactobacillus jensenii, which indicated that the antimicrobial effect was selective. Similar selectivity of acetic acid and/or its salt, dehydroacetic acid and/or its salt, and caprylic acid and/or its salt in inhibiting Lactococcus lactis and Lactobacillus had not been observed in a specific concentration range.

### Experimental example 2

1. Experimental objective: to observe the inhibition effects of solution compositions containing any two agents selected from a group consisting of 2-phenylethanol, sodium benzoate, sodium propionate, and sodium dehydroacetate.
2. Experimental method:
A. Experimental strains: Escherichia coli (ATCC25922) and Staphylococcus aureus (ATCC6538).
B. Experimental grouping: grouping the antimicrobial solution compositions into group-a and the group-b.
   Group-a: all of the antimicrobial solutions in group-a contained water and antimicrobial agents. Specific compatibilities of antimicrobial agents were shown in Table 7A; all of the antimicrobial solutions had a pH value of 4.3.
   Group-b: all of the antimicrobial solutions in group-b contained water, antimicrobial agents, and 6.5 percent (weight/volume) of maltose. Specific compatibilities of antimicrobial agents in group-b were referred to Table 7B, which were correspondingly identical to that of group-a. All of the antimicrobial solutions had pH values of 4.3.
C. Experimental method: the experiment was implemented basically according to the method in appendix C of GB 15979-2002.

3. Experimental result: as shown in Tables 7A and 7B, the inhibition ratio of each antimicrobial solution with a pH value of 4.3 was presented.

**Table 7A. Antimicrobial effects of the compatibility of two antimicrobial agents against Escherichia coli and Staphylococcus aureus**

| **Groups** | **pH** | **Antimicrobial agent (%)** | | | | **Escherichia coli** | | **Staphylococcus aureus** | |
|---|---|---|---|---|---|---|---|---|---|
| | | **2-Phen yletha nol** | **Sodiu m propio nate** | **Sodi um benz oate** | **Sodiu m dehydr oacetat e** | **Colony count after 20 minutes** | **Inhibiti on ratio** | **Colony count after 20 minute s** | **Inhibiti on ratio** |
| Antimicro bial solution 1 | 4.3 | **0.35** | 0 | 0 | 0 | 49 | 0.00% | 32 | 31.25% |
| Antimicro bial solution 2 | 4.3 | 0 | **0.5** | 0 | 0 | 47 | 0.00% | 37 | 18.75% |
| Antimicro bial solution 3 | 4.3 | 0 | 0 | **0.06** | 0 | 54 | 0.00% | 33 | 28.13% |
| Antimicro bial solution 4 | 4.3 | 0 | 0 | 0 | **0.02** | 32 | 17.95% | 48 | 0.00% |
| Antimicro bial solution 5 | 4.3 | **0.35** | **0.5** | 0 | 0 | 7 | 82.05% | 13 | 71.74% |
| Antimicro bial solution 6 | 4.3 | **0.35** | 0 | **0.06** | 0 | 2 | 94.12% | 1 | 96.77% |
| Antimicro bial solution 7 | 4.3 | **0.35** | 0 | 0 | **0.02** | 32 | 17.65% | 37 | 19.36% |
| Antimicro bial solution 8 | 4.3 | 0 | **0.5** | **0.06** | 0 | 31 | 20.0% | 24 | 46.88% |
| Antimicro bial solution 9 | 4.3 | 0 | **0.5** | 0 | **0.02** | 23 | 41.18% | 48 | 0.00% |
| Antimicro bial solution 10 | 4.3 | 0 | 0 | **0.06** | **0.02** | 42 | 0.00% | 37 | 19.35% |
| Positive Control | 7.0 | 0 | 0 | 0 | 0 | 39 | 0.00% | 46 | 0.00% |
| Negative control | 7.0 | 0 | 0 | 0 | 0 | 0 | 0.00% | 0 | 0.00% |

**Table 7B. Antimicrobial effects of the compatibility of two antimicrobial agent against Escherichia coli and Staphylococcus aureus**

| **Groups** | **pH** | **Malt ose (%)** | **Antimicrobial agent (%)** | | | | **Escherichia coli** | | **Staphylococcus aureus** | |
|---|---|---|---|---|---|---|---|---|---|---|
| | | | **2-Phe nyleth anol** | **Sodiu m propio nate** | **Sodi um benz oate** | **Sodiu m dehyd roacet ate** | **Colony count after 20 minutes** | **Antimi crobial rate** | **Colony count after 20 minutes** | **Antimi crobial rate** |
| Antimicro bial solution 1 | 4.3 | 6.5 | **0.35** | 0 | 0 | 0 | 53 | 0.00% | 44 | 8.70% |
| Antimicro bial solution 2 | 4.3 | 6.5 | 0 | **0.5** | 0 | 0 | 56 | 0.00% | 41 | 15.22% |
| Antimicro bial solution 3 | 4.3 | 6.5 | 0 | 0 | **0.06** | 0 | 61 | 0.00% | 40 | 17.39% |
| Antimicro bial solution 4 | 4.3 | 6.5 | 0 | 0 | 0 | **0.02** | 45 | 12.82% | 50 | 0.00% |
| Antimicro bial solution 5 | 4.3 | 6.5 | **0.35** | **0.5** | 0 | 0 | 12 | 76.92% | 16 | 67.39% |
| Antimicro bial solution 6 | 4.3 | 6.5 | **0.35** | 0 | **0.06** | 0 | 4 | 92.31% | 2 | 95.65% |
| Antimicro bial solution 7 | 4.3 | 6.5 | **0.35** | 0 | 0 | **0.02** | 43 | 17.95% | 41 | 15.22% |
| Antimicro bial solution 8 | 4.3 | 6.5 | 0 | **0.5** | **0.06** | 0 | 40 | 23.08% | 26 | 45.65% |
| Antimicro bial solution 9 | 4.3 | 6.5 | 0 | **0.5** | 0 | **0.02** | 27 | 48.72% | 51 | 0.00% |
| Antimicro bial solution 10 | 4.3 | 6.5 | 0 | 0 | **0.06** | **0.02** | 53 | 0.00% | 38 | 21.74% |
| Positive Control | 7 | 6.5 | 0 | 0 | 0 | 0 | 52 | 0.00% | 48 | 0.00% |
| Negative control | 7 | 0 | 0 | 0 | 0 | 0 | 0 | 0.00% | 0 | 0.00% |

4. Conclusion:
4.1 There was no significant difference between the inhibition ratio of two compatibilities that were identical in the content of antimicrobial agents and pH values, despite whether the 6.5 percent of maltose was contained or not.
4.2 When the pH value of the solution was 4.3, the compatibility of 0.35 percent (weight/volume) of 2-phenylethanol and 0.06 percent (weight/volume) of sodium benzoate had a synergistic antimicrobial effect against Escherichia coli and Staphylococcus aureus, the inhibition ratio thereof exceeded 50 percent. The compatibility of 0.35 percent (weight/volume) of 2-phenylethanol and 0.5 percent (weight/volume) of sodium propionate had a synergistic antimicrobial effect against Escherichia coli and Staphylococcus aureus, and the inhibition ratio thereof exceeded 50 percent.
4.3 When the pH value of the solution was 4.3, the compatibility of 0.35 percent (weight/volume) of 2-phenylethanol and 0.02 percent (weight/volume) of sodium dehydroacetate, the compatibility of 0.06 percent (weight/volume) of sodium benzoate and 0.02 percent (weight/volume) of sodium dehydroacetate, the compatibility of 0.5 percent (weight/volume) of sodium propionate and 0.02 percent (weight/volume) of sodium dehydroacetate, and the combination of 0.06 percent (weight/volume) of sodium benzoate and 0.5 percent (weight/volume) of sodium propionate thereof, exhibited a weaker antimicrobial effect respectively..
4.4 In conclusion, when the pH values of antimicrobial solution compositions were 4.3, the compatibility of 2-phenylethanol with sodium benzoate in specific concentration had a synergistic antimicrobial effect against Escherichia coli (ATCC 25922) and Staphylococcus aureus (ATCC 6538). The compatibility of 2-phenylethanol with sodium propionate in specific concentration had a synergistic antimicrobial effect against Escherichia coli (ATCC 25922) and Staphylococcus aureus (ATCC 6538).

However, any of the following compatibilities, i.e., 2-phenylethanol with sodium dehydroacetate, sodium propionate with sodium dehydroacetate, sodium propionate with sodium benzoate, and sodium dehydroacetate with sodium benzoate thereof, was less effective in inhibiting Escherichia coli and Staphylococcus aureus than that of the compatibility of 2-phenylethanol with sodium benzoate or with sodium propionate.

### Experimental example 3

1. Experimental objective: to observe the impact of pH on the inhibition effects of solution compositions containing any two agents selected from a group consisting of 2-phenylethanol, sodium benzoate, sodium propionate, and sodium dehydroacetate.
2. Experimental method:
A. Experimental strains: Escherichia coli (ATCC25922) and Staphylococcus aureus (ATCC 6538).
B. Experimental groups: as shown in Tables 8 and 9, each antimicrobial combination solution contained water and antimicrobial agents.
C. Experimental procedure: the experiment was implemented basically according to the method in appendix C of GB15979-2002.

3. Experimental result: as shown in Tables 8 and 9, the inhibition ratio of antimicrobial solutions with a pH value of 4.1 and the antimicrobial solutions with a pH value of 4.5 were presented:

**Table 8. The inhibitory effects of solutions containing two antimicrobial agents with pH value of 4.1 against Escherichia coli and Staphylococcus aureus**

| **Groups** | **pH** | **Antimicrobial agent (%)** | | | | **Escherichia coli** | | **Staphylococcus aureus** | |
|---|---|---|---|---|---|---|---|---|---|
| | | **2-Phenyl ethanol** | **Sodiu m propio nate** | **Sodiu m benzo ate** | **Sodiu m dehyd roacet ate** | **colony count after 20 minutes** | **Inhibitio n ratio** | **colony count after 20 minutes** | **Inhibiti on ratio** |
| Antimic robial solution 1 | 4.1 | **0.35** | **0.5** | 0 | 0 | 6 | 95.45% | 20 | 92.34% |
| Antimic robial solution 2 | 4.1 | **0.35** | 0 | **0.06** | 0 | 1 | 99.24% | 1 | 99.62% |
| Antimic robial solution 3 | 4.1 | **0.35** | 0 | 0 | **0.02** | 94 | 28.79% | 133 | 49.04% |
| Antimic robial solution 4 | 4.1 | 0 | **0.5** | **0.06** | 0 | 122 | 7.58% | 184 | 29.50% |
| Antimicrobial solution 5 | 4.1 | 0 | **0.5** | 0 | **0.02** | 68 | 48.48% | 245 | 6.13% |
| Antimic robial solution 6 | 4.1 | 0 | 0 | **0.06** | **0.02** | 73 | 44.70% | 220 | 15.71% |
| Positive Control | 7.0 | 0 | 0 | 0 | 0 | 132 | 0.00% | 261 | 0.00% |
| Negative control | 7.0 | 0 | 0 | 0 | 0 | 0 | 0 | 0 | 0 |

**Table 9. The inhibitory effects of solutions containing two antimicrobial agents with a pH value of 4.5 against Escherichia coli and Staphylococcus aureus**

| **Groups** | **pH** | **Antimicrobial agent (%)** | | | | **Escherichia coli** | | **Staphylococcus aureus** | |
|---|---|---|---|---|---|---|---|---|---|
| | | **2-Phen yletha nol** | **Sodiu m propio nate** | **Sodiu m benzo ate** | **Sodiu m dehyd roacet ate** | **Colony count after 20 minutes** | **Inhibitio n ratio** | **Colony count after 20 minutes** | **Inhibiti on ratio** |
| Antimicr obial solution 1 | 4.5 | **0.35** | **0.5** | 0 | 0 | 30 | 34.78% | 61 | 12.86% |
| Antimicr obial solution 2 | 4.5 | **0.35** | 0 | **0.06** | 0 | 39 | 15.22% | 61 | 12.86% |
| Antimicr obicsoluti on 3 | 4.5 | **0.35** | 0 | 0 | **0.02** | 53 | 0.00% | 84 | 0.00% |
| Antimicr obial solution 4 | 4.5 | 0 | **0.5** | **0.06** | 0 | 69 | 0.00% | 75 | 0.00% |
| Antimicr obial solution 5 | 4.5 | 0 | **0.5** | 0 | **0.02** | 41 | 10.87% | 48 | 31.43% |
| Antimicr obial solution 6 | 4.5 | 0 | 0 | **0.06** | **0.02** | 78 | 0.00% | 77 | 0.00% |
| Positive Control | 7.0 | 0 | 0 | 0 | 0 | 46 | 0.00% | 70 | 0.00% |
| Negative control | 7.0 | 0 | 0 | 0 | 0 | 0 | 0 | 0 | 0 |

4. Conclusion:
Compared with the previous experimental example 2, the pH values of the antimicrobial solutions of this experimental example 3 were adjusted to 4.1 and 4.5, while the concentration of each antimicrobial agent was not changed, so as to observe the impact of pH on the inhibition effects of solution compositions.

When the pH value of the solution compositions was 4.1, the combination solution of 0.35 percent (weight/volume) of 2-phenylethanol and 0.5 percent (weight/volume) of sodium propionate, and the combination solution of 0.35 percent (weight/volume) of 2-phenylethanol and 0.06 percent (weight/volume) of sodium benzoate, respectively had a stronger antimicrobial effect against Escherichia coli (ATCC 25922) and Staphylococcus aureus (ATCC 6538), and the inhibition ratio of these combination solutions were higher than that of the corresponding solutions with pH value of 4.3. However, when the pH value of the solution was 4.5, the combination solution of 0.35 percent (weight/volume) of 2-phenylethanol and 0.5 percent (weight/volume) of sodium propionate, and the combination solution of 0.35 percent (weight/volume) of 2-phenylethanol and 0.06 percent (weight/volume) of sodium benzoate respectively had a weaker antimicrobial effect against Escherichia coli and Staphylococcus aureus.

However, even if the pH value of the solution was 4.1, the inhibition effects of all the combinations such as the combination of 2-phenylethanol and sodium dehydroacetate, the combination of sodium benzoate and sodium dehydroacetate, the combination of sodium propionate and sodium dehydroacetate, and the combination of sodium benzoate and sodium propionate, was still weak, and the inhibition ratio of these combinations were lower than 50 percent respectively.

To sum up and combined with the results of experimental example 2, it was shown that the combination of 2-phenylethanol and sodium benzoate, and the combination of 2-phenylethanol and sodium propionate of specific concentration were synergistic in inhibiting against Escherichia coli and Staphylococcus aureus.The pH value had significant influence on the inhibition effects of the combination solution of 2-phenylethanol and sodium benzoate, and the combination solution of 2-phenylethanol and sodium propionate. In the range from 4.1 to 4.5, the solution with a lower pH value had a stronger synergistic inhibition effect, and the solution with a higher pH value had a weaker synergistic inhibition effect.

### Experimental example 4

1. Experimental objective: to observe the inhibition effects of the antimicrobial gels containing any two agents selected from a group consisting of 2-phenylethanol, sodium benzoate, sodium propionate, and sodium dehydroacetate..
2. Experimental method:
A. Experimental strains: Escherichia coli (ATCC25922) and Staphylococcus aureus (ATCC 6538).
B. Experimental groups: as shown in Table 10, each antimicrobial combination gel contained water, antimicrobial agent, and xanthan gum,.
C. Experimental procedure: the experiment was implemented basically according to the method in appendix C of GB 15979-2002.

3. Experimental result: as shown in Table 10, the inhibition ratio of each antimicrobial gel with pH value 4.3 was presented:

**Table 10. The inhibitory effects of the gels containing two antimicrobial agents against Escherichia coli and Staphylococcus aureus**

| **Groups** | **pH** | **Xant han gum( %)** | **Antimicrobial agent (%)** | | | | **Escherichia coli** | | **Staphylococcus aureus** | |
|---|---|---|---|---|---|---|---|---|---|---|
| | | | **2-Phen yletha nol** | **Sodiu m propio nate** | **Sodiu m benzo ate** | **Sodiu m dehydr oacetat e** | **Colony count after 20 minute s** | **Inhibiti on ratio** | **Colony count after 20 minute s** | **Inhibitio n ratio** |
| Antimicro bial solution 1 | 4.3 | 1.0 | **0.375** | 0 | 0 | 0 | 127 | 0.00% | 95 | 16.67% |
| Antimicro bial solution 2 | 4.3 | 1.0 | 0 | **0.55** | 0 | 0 | 150 | 0.00% | 67 | 41.23% |
| Antimicro bial solution 3 | 4.3 | 1.0 | 0 | 0 | **0.06** | 0 | 100 | 10.71% | 130 | 0.00% |
| Antimicro bial solution 4 | 4.3 | 1.0 | 0 | 0 | 0 | **0.02** | 100 | 10.71% | 138 | 0.00% |
| Antimicro bial solution 5 | 4.3 | 1.0 | **0.375** | **0.55** | 0 | 0 | 29 | 74.11% | 33 | 71.05% |
| Antimicro bial solution 6 | 4.3 | 1.0 | **0.375** | 0 | **0.06** | 0 | 66 | 41.07% | 56 | 50.88% |
| Antimicro bial solution 7 | 4.3 | 1.0 | **0.375** | 0 | 0 | **0.02** | 80 | 28.57% | 114 | 0.00% |
| Antimicro bial solution 8 | 4.3 | 1.0 | 0 | **0.55** | **0.06** | 0 | 86 | 23.21% | 116 | 0.00% |
| Antimicro bial solution 9 | 4.3 | 1.0 | 0 | **0.55** | 0 | **0.02** | 92 | 17.86% | 110 | 3.51% |
| Antimicro bial solution 10 | 4.3 | 1.0 | 0 | 0 | **0.06** | **0.02** | 78 | 30.36% | 161 | 0.00% |
| Positive Control | 7 | 1.0 | 0 | 0 | 0 | 0 | 112 | 0.00% | 114 | 0.00% |
| Negative control | 7 | 0 | 0 | 0 | 0 | 0 | 0 | 0.00% | 0 | 0.00% |

4. Conclusion:
4.1 When the pH value of the antimicrobial gel was 4.3, 0.375 percent (weight/volume) of 2-phenylethanol combined with 0.06 percent (weight/volume) of sodium benzoate had a synergistic antimicrobial effect against Escherichia coli and Staphylococcus aureus, and the inhibition ratio of the combination against Staphylococcus aureus exceeded 50 percent. 0.375 percent (weight/volume) of 2-phenylethanol combined with 0.55 percent (weight/volume) of sodium propionate had a synergistic antimicrobial effect against Escherichia coli and Staphylococcus aureus, and both of the inhibition ratio against Escherichia coli and on Staphylococcus aureus exceeded 50 percent.
4.2 When the pH value of antimicrobial gel was 4.3, the antimicrobial effect of the following combinations was not as good as that of the combination of 2-phenylethanol with sodium benzoate or with sodium propionate: the combination of 0.375 percent (weight/volume) of 2-phenylethanol and 0.02 percent (weight/volume) of sodium dehydroacetate, the combination of 0.06 percent (weight/volume) of sodium benzoate and 0.02 percent (weight/volume) of sodium dehydroacetate, the combination of 0.55 percent (weight/volume) of sodium propionate and 0.02 percent (weight/volume) of sodium dehydroacetate, and the combination of 0.06 percent (weight/volume) of sodium benzoate and 0.55 percent (weight/volume) of sodium propionate.

In conclusion, the inhibition effects of the antimicrobial gels as revealed in the experimental example further demonstrated that the combination of 2-phenylethanol and sodium propionate, and the combination of 2-phenylethanol and sodium benzoate had a strong synergistic antimicrobial effect against Escherichia coli and/or Staphylococcus aureus.

### Experimental example 6

1. Experimental objective: to observe inhibition effects of solution compositions containing three antimicrobial agents.
2. Experimental method:
A. Experimental strains: Escherichia coli (ATCC25922) and Staphylococcus aureus (ATCC 6538).
B. Experimental groups: as shown in Table 15, each antimicrobial solution contained water and antimicrobial agents,.
C. Experimental procedure: the experiment was implemented basically according to the method in appendix C of GB 15979-2002.

3. Experimental result: as shown in Table 15, the inhibition effects of antimicrobial solutions with pH value of 4.3 were presented:

**Table 15.The inhibitory effects of the combination solutions containing three antimicrobial agents against Escherichia coli and Staphylococcus aureus**

| **Groups** | **pH** | **Antimicrobial agent (%)** | | | | **Escherichia coli** | | **Staphylococcus aureus** | |
|---|---|---|---|---|---|---|---|---|---|
| | | **2-pheny lethanol** | **Sodiu m propio nate** | **Sodiu m benzo ate** | **Sodiu m dehyd roacet ate** | **Colony count after 20 minute s** | **Inhibiti on ratio** | **Colony count after 20 minutes** | **Inhibiti on ratio** |
| Antimicr obial solution 1 | 4.3 | **0.35** | **0.5** | **0.05** | **0.02** | 0 | 100.00% | 0 | 100.00% |
| Antimicr obial solution 2 | 4.3 | 0 | **0.5** | **0.05** | **0.02** | 23 | 54.00% | 27 | 0.00% |
| Antimicr obial solution 3 | 4.3 | **0.35** | 0 | **0.05** | **0.02** | 3 | 94.00% | 10 | 62.96% |
| Antimicr obial solution 4 | 4.3 | **0.35** | **0.5** | **0.05** | 0 | 0 | 100.00% | 0 | 100.00% |
| Antimicr obial solution 5 | 4.3 | **0.35** | **0.5** | 0 | **0.02** | 0 | 100.00% | 14 | 48.15% |
| Positive Control | 7.0 | 0 | 0 | 0 | 0 | 50 | 0.00% | 27 | 0.00% |
| Begative control | 7.0 | 0 | 0 | 0 | 0 | 0 | 0 | 0 | 0 |

4. Conclusion:
In the present experimental example, there were four combination solutions containing three agents, among which the combination solution (pH 4.3) containing "0.35 percent (weight/volume) of 2-phenylethanol, 0.5 percent (weight/volume) of sodium propionate, and 0.05 percent (weight/volume) of sodium benzoate" was most effective. It's inhibition ratio against Escherichia coli and Staphylococcus aureus were 100 percent respectively, which is significantly higher than that of the combination solution of 0.35 percent (weight/volume) of 2-phenylethanol and 0.5 percent (weight/volume) of sodium propionate, and the combination of 0.35 percent (weight/volume) of 2-phenylethanol and 0.05 percent (weight/volume) of sodium benzoates (pH 4.3). However, it was comparable to that of the combination solution containing 0.35 percent (weight/volume) of 2-phenylethanol, 0.5 percent (weight/volume) of sodium propionate, 0.05 percent (weight/volume) of sodium benzoate, and 0.02% sodium dehydroacetate.

### Experimental example 7

1. Experimental objective: to observe the antimicrobial effect of the combination solutions of lower pH value, which contained 2-phenylethanol, sodium propionate, and sodium benzoate.
2. Experimental method:
A. Experimental strains: Escherichia coli (ATCC25922) and Staphylococcus aureus (ATCC 6538).
B. Experiment groups: as shown in Table 16, each antimicrobial solution contained water and antimicrobial agents,.
C. Experimental procedure: the experiment was implemented basically according to the method in appendix C of GB 15979-2002.

3. Experimental result: as shown in Table 16, the inhibition effects of the antimicrobial solutions (pH 4.1) containing different components were presented.

**Table 16. The inhibitory effects of combinations solutions containing three antimicrobial agents against Escherichia coli and Staphylococcus aureus**

| **Groups** | **pH** | **Antimicrobial agent (%)** | | | | **Escherichia coli** | | **Staphylococcus aureus** | |
|---|---|---|---|---|---|---|---|---|---|
| | | **2-Phen ylethan ol** | **Sodiu m propio nate** | **Sodiu m benzo ate** | **Sodium dehydr oacetat e** | **Colony count after 20 minute s** | **Inhibiti on ratio** | **Colony count after 20 minutes** | **Inhibiti on ratio** |
| Antimicr obial solution 1 | 4.1 | **0.35** | **0.5** | **0.05** | **0.02** | 0 | 100.00% | 0 | 100.00% |
| Antimicr obial solution 2 | 4.1 | 0 | **0.5** | **0.05** | **0.02** | 56 | 0.00% | 21 | 59.62% |
| Antimicr obial solution 3 | 4.1 | **0.35** | 0 | **0.05** | **0.02** | 5 | 86.49% | 0 | 100.00% |
| Antimicr obial solution 4 | 4.1 | **0.35** | **0.5** | **0.05** | 0 | 0 | 100.00% | 0 | 100.00% |
| Antimicr obial solution 5 | 4.1 | **0.35** | **0.5** | 0 | **0.02** | 17 | 54.05% | 0 | 100.00% |
| Positive Control | 7.0 | 0 | 0 | 0 | 0 | 37 | 0.00% | 52 | 0.00% |
| Negative control | 7.0 | 0 | 0 | 0 | 0 | 0 | 0 | 0 | 0 |

4. Conclusion:
The antimicrobial effect against Staphylococcus aureus of combination solutions containing three antimicrobial agents (pH 4.1) were stronger than that of solutions containing identical antimicrobial agents but with a pH value of 4.3. The combination solution containing :0.35 percent (weight/volume) of 2-phenylethanol, 0.5 percent (weight/volume) of sodium propionate, and 0.05 percent (weight/volume) of sodium benzoate showed the strongest inhibitory action, of which the inhibition ratio were 100 percent for aginst Escherichia coli as well as for against Staphylococcus aureus.

The inhibition ratio of the combination solution containing 2-phenylethanol, sodium propionate, and sodium benzoate was also significantly higher than that of the combination solution containing 0.35 percent (weight/volume) of 2-phenylethanol and 0.5 percent (weight/volume) of sodium propionate, and the combination of 0.35 percent (weight/volume) of 2-phenylethanol and 0.05 percent (weight/volume) of sodium benzoates (pH 4.1). It was comparable to that of the combination solution containing 0.35 percent (weight/volume) of 2-phenylethanol, 0.5 percent (weight/volume) of sodium propionate, 0.05 percent (weight/volume) of sodium benzoate, and 0.02% sodium dehydroacetate (pH 4.1).

The antimicrobial effect of the combination solution containing 0.5 percent (weight/volume) of sodium propionate, 0.05 percent (weight/volume) of sodium benzoate, and 0.02 percent (weight/volume) of sodium dehydroacetate was the weakest. It had little antimicrobial effect against Escherichia coli while the inhibition ratio against Staphylococcus aureus was 59.62 percent.

### Experimental Example 8

1. Experimental objective: to observe inhibition effects of gel compositions containing three antimicrobial agents.
2. Experimental method:
A. Experimental strains: Escherichia coli (ATCC25922) and Staphylococcus aureus (ATCC 6538).
B. Experimental groups: as shown in Table 17, each antimicrobial gel group contained water, antimicrobial agents, and xanthan gum.
C. Experimental procedure: the experiment was implemented basically according to the method in appendix C of GB 15979-2002.

3. Experimental result: as shown in Table 17, the inhibition effects of the antimicrobial gels containing different antimicrobial agents (pH 4.1) were presented.

**Table 17. The inhibitory effects of combination gels containing three antimicrobial agents against Escherichia coli and Staphylococcus aureus**

| **Groups** | **pH** | **Xant han gum( %)** | **Antimicrobial agent (%)** | | | | **Escherichia coli** | | **Staphylococcus aureus** | |
|---|---|---|---|---|---|---|---|---|---|---|
| | | | **2-Phe nyleth anol** | **Sodiu m propio nate** | **Sodi um benz oate** | **Sodiu m dehydr oacetat e** | **Colony count after 20 minutes** | **Inhibiti on ratio** | **Colony count after 20 minutes** | **Inhibiti on ratio** |
| Antimicr obial solution 1 | 4.1 | 1.0 | **0.4** | **0.55** | **0.07** | **0.01** | 32 | 89.08% | 28 | 89.86% |
| Antimicr obial solution 2 | 4.1 | 1.0 | **0.4** | **0.55** | **0.07** | 0 | 1 | 99.66% | 24 | 91.30% |
| Antimicr obial solution 3 | 4.1 | 1.0 | **0.4** | **0.55** | 0 | **0.01** | 147 | 49.83% | 51 | 81.52% |
| Antimicr obial solution 4 | 4.1 | 1.0 | 0.4 | 0 | **0.07** | **0.01** | 1 | 99.66% | 30 | 89.13% |
| Antimicr obial solution 5 | 4.1 | 1.0 | 0 | **0.55** | **0.07** | **0.01** | 220 | 24.91% | 208 | 24.64% |
| Positive Control | 7 | 1.0 | 0 | 0 | 0 | 0 | 293 | 0.00% | 276 | 0.00% |
| Negative control | 7 | 1.0 | 0 | 0 | 0 | 0 | 0 | 0.00% | 0 | 0.00% |

4. Conclusion:
Among the combination gels (pH 4.1), the gel containing 0.4 percent (weight/volume) of 2-phenylethanol, 0.55 percent (weight/volume) of sodium propionate, and 0.07 percent (weight/volume) of sodium benzoatev showed the strongest inhibition effects. The inhibition ratio against Escherichia coli and Staphylococcus aureus were 99.66 percent and 91.30 percent respectively. The antimicrobial effect of the gel containing sodium propionate, sodium benzoate, and sodium dehydroacetate, which did not contain 2-phenylethanol, was the weakest, and the inhibition ratio against Escherichia coli and Staphylococcus aureus were both less than 50 percent.

### Experimental example 10

1. Experimental objective: to observe the inhibition effects of the antimicrobial agent combination of the present disclosure against the growth of Escherichia coli and Staphylococcus aureus.
2. Experimental method:
   (1). Experimental material:
      a. Experimental strains, three strains of Escherichia coli and three strains of Staphylococcus aureus, which were all isolated from the vaginal secretion of patients with vaginitis.
      b. Enrichment broth, which contained 1 percent of yeast extract powder, 9 percent of sucrose, 0.025 percent of manganese sulfate (MnSO₄.4H₂O), 0.058 percent of magnesium sulfate (MgSO₄.7H₂O), and 0.9 percent of lactic acid, the pH value of the broth was adjusted to 4.8, and the enrichment broth was sterilized for later use;
   (2). Experimental groups: the requirements for aseptic operation technique were strictly followed to prepare the sterile test tubes:
      a. sodium benzoate group: each test tube contained 5 milliliters of enrichment broth,and was added in 0.05 percent (weight/volume) sodium benzoate ;
      b. 2-phenylethanol group: each test tube contained 5 milliliters of enrichment broth, and was added in 0.05 percent (weight/volume) of 2-phenylethanol;
      c. sodium propionate group: each test tube contained 5 milliliters of enrichment broth, and was added in 0.25 percent (weight/volume) of sodium propionate;
      d. "a group of the combinations of sodium benzoate and sodium propionate": each test tube contained 5 milliliters of enrichment broth, and was added in 0.05 percent (weight/volume) of sodium benzoate and 0.25 percent (weight/volume) of sodium propionate;
      e. "a group of the combinations of sodium benzoate and 2-phenylethanol": each test tube contained 5 milliliters of enrichment broth, and was added in 0.05 percent (weight/volume) of sodium benzoate and 0.05 percent (weight/volume) of 2-phenylethanol;
      f. "a group of the combinations of sodium propionate and 2-phenylethanol": each test tube contained 5 milliliters of enrichment broth, and was added in 0.25 percent (weight/volume) of sodium propionate and 0.05 percent (weight/volume) of 2-phenylethanol;
      g. "a group of the combinations of sodium benzoate, sodium propionate and 2-phenylethanol": each test tube contained 5 milliliters of enrichment broth, and was added in 0.05 percent (weight/volume) of sodium benzoate, 0.25 percent (weight/volume) of sodium propionate, and 0.05 percent (weight/volume) of 2-phenylethanol;
      h. positive control: one tube that contained 5 milliliters of enrichment broth without adding in any of the 2-phenylethanol, sodium benzoate, and sodium propionate, but inoculated the suspension of Escherichia coli or Staphylococcus aureus.
      i. negative control: one tube that contained 5 milliliters of enrichment broth without adding in any of the 2-phenylethanol, sodium benzoate, and sodium propionate, and not inoculated the suspension of Escherichia coli or Staphylococcus aureus.
   (3). Experimental procedure:
      The requirements for aseptic operation technique were strictly followed to prepare the bacterial suspensions. The colonies of Escherichia coli and Staphylococcus aureus were diluted with physiological saline respectively to prepare bacterial suspension. The turbidities of the Escherichia coli suspension and the Staphylococcus aureus suspension were adjusted to 0.5MCF (about 10⁸CFU/ml) by a turbidimeter. 1 milliliter of the 0.5MCF bacterial suspensions were added into 9 milliliters of the enrichment broth respectively, so each bacterial solution was diluted for ten times to contain 10⁷CFU/ml of bacterial organisms.

The requirements for aseptic operation technique were strictly followed to take 100 microliters of the prepared bacterial suspensions respectively, and to add the bacterial suspensions into the aforesaid test tubes from a to h as well as positive control tube. No bacterial suspension was added into the negative control tube i.

The above tubes were cultured in a biochemical incubator at 37°C for 24 hours, then turbidity changes of the test tube solutions were observed and recorded.

### 3. Experimental result:

As shown in table 22A:

**Table 22A. Effects of antimicrobial agent solutions against the growth of Escherichia coli and Staphylococcus aureus**

| **Bacterial strain** | **Escherichia coli** | **Staphylococcus aureus** |
|---|---|---|
| | **1 2 3** | **1 2 3** |
| **a. 0.05 % sodium benzoate** | + + + | + + + |
| **b. 0.05% 2-phenylethanol** | + + + | + + + |
| **c. 0.25% sodium propionate** | + + + | + + + |
| **d. 0.05% sodium benzoate and 0.25% sodium propionate** | + + + | + + + |
| **e. 0.05% sodium benzoate and 0.05% 2-phenylethanol** | - + + | + + + |
| **f. 0.25% sodium propionate and 0.05% 2-phenylethanol** | - + + | + - + |
| **g. 0.05% sodium benzoate and 0.25% sodium propionate and 0.05% 2-phenylethanol** | - + - | + - - |
| **h. positive control** | + + + | + + + |
| **i. negative control** | - - - | - - - |

**Remark: "+" means the growth of bacteria; "-" means no growth of bacteria.**

### 4. Conclusion:

(1) In the experimental example, no one of the three antimicrobial agents, i.e., 0.05 percent (weight/volume) of sodium benzoate, 0.05 percent (weight/volume) of 2-phenylethanol, and 0.25 percent (weight/volume) of sodium propionate, could inhibit the growth of Escherichia coli and Staphylococcus aureus respectively.
(2) In the experimental example, the combination of "0.05 percent (weight/volume) of sodium benzoate and 0.25 percent (weight/volume) of sodium propionate" had no significant inhibition effect against the growth of Escherichia coli and Staphylococcus aureus.
(3) In the experimental example, the growth of one-third of the Escherichia coli strains were inhibited by the combination of "0.05 percent (weight/volume) of sodium benzoate and 0.05 percent (weight/volume) of 2-phenylethanol"; but the growth of Staphylococcus aureus strains were not inhibited by the combination of "0.05 percent (weight/volume) of sodium benzoate and 0.05 percent (weight/volume) of 2-phenylethanol". The growth of one-third of the Escherichia coli strains and one-third of the Staphylococcus aureus strains were inhibited by the combination of "0.25 percent (weight/volume) of sodium propionate and 0.05 percent (weight/volume) of 2-phenylethanol".
(4) In the experimental example, the growth of two-thirds Escherichia coli strains and two-thirds Staphylococcus aureus strains were inhibited by the combination of "0.05 percent (weight/volume) of 2-phenylethanol, 0.05 percent (weight/volume) of sodium benzoate, and 0.25 percent (weight/volume) of sodium propionate". It was suggested that the combination of the three antimicrobial agents had a synergistic antimicrobial effect against Escherichia coli and Staphylococcus aureus.

### Experimental example 12

1. Experimental objective: to observe the modulating effect of the gel containing 9.0 percent (weight/volume) of maltose, 0.05 percent (weight/volume) of 2-phenylethanol, and 0.075 percent (weight/volume) of sodium benzoate on vaginal flora of rhesus macaque.
2. Experimental method:
A. Preparation of gel: according to the preparation method of the present disclosure, the gel was prepared by using 9.0 percent (weight/volume) of maltose, 0.05 percent (weight/volume) of 2-phenylethanol, 0.075 percent (weight/volume) of sodium benzoate, and 2.2 percent (weight/volume) of Xanthan gum. The pH value of the gel was adjusted to 3.0.
B. Animals: one adult female rhesus macaque. The vaginal secretion pH >5.4. Vaginal smear examination showed dominant Gram-negative rods (G^{-b}) and with a number of Gram-negative cocci (G^{-c}) and Gram-positive cocci (G^{+c}). Large Gram-positive rods (G^{+b}) were rare.
C. Animal treatment: 0.5ml of the gel was intravaginally administered, once a day for consecutive 5 days. Vaginal secretion was sampled on the 6th day. The pH value of vaginal secretion was tested, and the vaginal smears were microscopically examined after Gram-staining. The results were compared with those before treatment.

3. Experimental result
As shown in table 23A:

**Table 23A. Effects of antimicrobial gel on vaginal flora of rhesus macaque**

| Animal No. | Before administration | | 1 Day after the end of administration | |
|---|---|---|---|---|
| | The vaginal secretion pH | The morphology and quantity of bacteria | The vaginal secretio n pH | The morphology and quantity of bacteria |
| 23736 | 5.4 | G^{-b}+++, G^{+c}+, G^{-c}+ | 3.8 | G^{+b}+++ |

4. Conclusion

The administration of the gel of the present disclosure, which comprised 9.0 percent (weight/volume) of maltose, 0.05 percent (weight/volume) of 2-phenylethanol, and 0.075 percent (weight/volume) of sodium benzoate, could restore vaginal flora of rhesus macaque to normal by reducing Gram-negative rods and increasing Gram-positive rods. The vaginal secretion pH could also be reduced.

### Experimental example 13

1. Experimental objective: to observe the modulating effect of the gel containing 5.5 percent (weight/volume) of maltose, 1.0 percent (weight/volume) of isomaltulose, 0.05 percent (weight/volume) of 2-phenylethanol, and 0.15 percent (weight/volume) of sodium benzoate on the BV-type flora of rhesus macaque.
2. Experimental method:
A. Preparation of gel: according to the preparation method of the present disclosure, the gel was prepared by using 5.5 percent (weight/volume) of maltose, 1.0 percent (weight/volume) of isomaltulose, 0.05 percent (weight/volume) of 2-phenylethanol, 0.15 percent (weight/volume) of sodium benzoate, and 2.15 percent (weight/volume) of Xanthan gum. The pH value of the gel was adjusted to 3.3.
B. Animals: two adult female rhesus macaques. The pH values of vaginal secretion of the two rhesus macaques were both 5.4. Vaginal smear examination showed dominant Gram-negative rods. Large Gram-positive rods were rare.
C. Animal treatment: 0.5ml of the gel was intravaginally administered, once a day for consecutive 5 days. The vaginal secretion was sampled on the 6th day. The pH values of vaginal secretion were tested, and the vaginal smears were microscopically examined after Gram-staining. The results were compared with those before treatment.

3. Experimental result
As shown in Table 23B:

**Table 23B. Effects of antimicrobial gel on vaginal flora of rhesus macaque**

| Animal No. | Before administration | | 1 Day after the end of administration | |
|---|---|---|---|---|
| | The vaginal secretion pH | The morphology and quantity of bacteria | The vaginal secretion pH | The morphology and quantity of bacteria |
| 3966 | 5.4 | G^{-b}+++ | <3.8 | G^{+b}+++ |
| 1204156 | >5.4 | G^{-b}+++ | 5.4 | G^{-b}+++, WBC+ |

4. Conclusion

The administration of the gel of the present disclosure, which comprised 5.5 percent (weight/volume) of maltose, 1.0 percent (weight/volume) of isomaltulose, 0.05 percent (weight/volume) of 2-phenylethanol, and 0.15 percent (weight/volume) of sodium benzoate, restored vaginal BV-type flora of one rhesus macaque to normal. The vaginal secretion pH values of this rhesus macaque could also be reduced.

### Experimental example 14

1. Experimental objective: to observe the modulating effect of the gel containing 6.5 percent (weight/volume) of sucrose, 0.10 percent (weight/volume) of 2-phenylethanol, and 0.05percent (weight/volume) of sodium benzoate on the vaginal flora of rhesus macaque.
2. Experimental method:
   A. Preparation of gel: according to the preparation method of the present disclosure, the gel was prepared by using 6.5 percent (weight/volume) of sucrose, 0.10 percent (weight/volume) of 2-phenylethanol, 0.05 percent (weight/volume) of sodium benzoate, and 2.15 percent (weight/volume) of Xanthan gum. The pH value of the gel was adjusted to 3.8.
   B. Animals: two adult female rhesus macaques. The pH values of vaginal secretion of the two rhesus macaques were both 5.4. Vaginal smear examination showed dominant Gram-negative rods. Large Gram-positive rods were rare.
   C. Animal treatment: 0.5ml of the gel was intravaginally administered, once a day for consecutive 5 days. The vaginal secretion was sampled on the 6th day. The pH values of vaginal secretion were tested. The vaginal smears were microscopically examined after Gram-staining. The results were compared with those before treatment.

As shown in Table 24:

**Table 24. Effects of antimicrobial gel on vaginal flora of rhesus macaque**

| Animal No. | Before administration | | 1 Day after the end of administration | |
|---|---|---|---|---|
| | The vaginal secretio n pH | The morphology and quantity of bacteria | The vaginal secretio n pH | The morphology and quantity of bacteria |
| 3966 | 5.4 | G^{-b}+++ | 3.8 | G^{+b}+++ |
| 13046 | 5.4 | G^{-b}+++, G^{+c}+++, few G+b | <3.8 | G^{+b}+++ mostly existed in epithelial cells |

### 4. Conclusion

The administration of the gel of the present disclosure, which comprised 6.5 percent (weight/volume) of sucrose, 0.10 percent (weight/volume) of 2-phenylethanol, and 0.05 percent (weight/volume) of sodium benzoate, could restore vaginal flora of rhesus macaques to normal. The vaginal secretion pH values of rhesus macaques could also be reduced.

### Experimental example 15

1. Experimental objective: to observe the modulating effect of the gel containing 1.0 percent (weight/volume) of glucose, 0.30 percent (weight/volume) of 2-phenylethanol, and 0.085 percent (weight/volume) of sodium benzoate on the vaginal flora of rhesus macaque.
2. Experimental method:
A. Preparation of gel: according to the preparation method of the present disclosure, the gel was prepared by using 1.0 percent (weight/volume) of glucose, 0.30 percent (weight/volume) of 2-phenylethanol, 0.085 percent (weight/volume) of sodium benzoate, and 2.15 percent (weight/volume) of Xanthan gum. The pH value of the gel was adjusted to 3.8.
B. Animals: one adult female rhesus macaque. The pH value of the vaginal secretion was 5.4. The vaginal smear examination showed dominant Gram-negative rods. Large Gram-positive rods were rare.
C. Animal treatment: 0.5ml of the gel was intravaginally administered, once a day for consecutive 5 days. The vaginal secretion was sampled on the 6th day. The pH value of vaginal secretion was tested, and the vaginal smear was microscopically examined after Gram-staining. The results were compared with those before treatment.

3. Experimental result
As shown in table 25:

**Table 25. Effects of antimicrobial gel combination on vaginal flora of rhesus macaque**

| Animal No. | Before administration | | 1 Day after the end of administration | |
|---|---|---|---|---|
| | The vaginal secretion pH | The morphology and quantity of bacteria | The vaginal secretion pH | The morphology and quantity of bacteria |
| 13284 | 5.4 | G^{-b}+++, few G^{+b} | 3.8 to 4.1 | G^{+b}+++, G^{+c}+, WBC+ |

4. Conclusion

The administration of the gel of the present disclosure, which comprised 1.0 percent (weight/volume) of glucose, 0.30 percent (weight/volume) of 2-phenylethanol, and 0.085 percent (weight/volume) of sodium benzoate, could restore vaginal flora of rhesus macaques to normal. The vaginal secretion pH values of rhesus macaques could also be reduced.

### Experimental example 16

1. Experimental objective: to observe the modulating effect of the gel containing 5.0 percent (weight/volume) of fructose, 0.40percent (weight/volume) of 2-phenylethanol, and 0.09 percent (weight/volume) of sodium benzoate on the vaginal flora of rhesus macaque.
2. Experimental method:
A. Preparation of gel: according to the preparation method of the present disclosure, the gel was prepared by using 5.0 percent (weight/volume) of fructose, 0.4 percent (weight/volume) of 2-phenylethanol, 0.09 percent (weight/volume) of sodium benzoate, and 2.15 percent (weight/volume) of Xanthan gum. The pH value of the gel was adjusted to 4.1
B. Animals: two adult female rhesus macaques. The pH values of the vaginal secretion were 5.4. The vaginal smear examination showed dominant Gram-negative rods. Large Gram-positive rods were rare
C. Animal treatment: 0.5ml of the gel was intravaginally administered, once a day for consecutive 5 days. The vaginal secretion was sampled on the 6th day. The pH values of vaginal secretion were tested. The vaginal smears were microscopically examined after Gram-staining. The results were compared with those before treatment.

3. Experimental result
As shown in table 26:

**Table 26 Effects of antimicrobial gel combination on vaginal flora of rhesus macaque**

| Animal No. | Before administration | | 1 Day after the end of administration | |
|---|---|---|---|---|
| | The vaginal secretio n pH | The morphology and quantity of bacteria | The vaginal secretion pH | The morphology and quantity of bacteria |
| 13278 | 5.4 | G^{-b}+++, G^{+c}++ | <3.8 | G^{+b}++ |
| 6058 | 5.4 | G^{-b}+++, G^{+c}++, G^{+b}+, WBC 0~+ | 4.4 | G^{+b}++, WBC++ |

4. Conclusion

The administration of the gel of the present disclosure, which comprised 5.0 percent (weight/volume) of fructose, 0.4 percent (weight/volume) of 2-phenylethanol, and 0.09 percent (weight/volume) of sodium benzoate, could restore vaginal flora of rhesus macaques to normal. The vaginal secretion pH values of rhesus macaques could also be reduced.

### Experimental example 17

1. Experimental objective: to observe the modulating effect of the gel containing 4.0 percent (weight/volume) of mannose, 0.10 percent (weight/volume) of 2-phenylethanol, and 0.35 percent (weight/volume) of sodium propionate on the vaginal flora of rhesus macaque.
2. Experimental method:
A. Preparation of gel: according to the preparation method of the present disclosure, the gel was prepared by using 4.0 percent (weight/volume) of mannose, 0.10 percent (weight/volume) of 2-phenylethanol, 0.35 percent (weight/volume) of sodium propionate, and 2.15 percent (weight/volume) of Xanthan gum. The pH value of the gel was adjusted to 3.5.
B. Animals: one adult female rhesus macaque. The pH value of the vaginal secretion was 5.4. Vaginal smear examination showed dominant Gram-negative rods. Large Gram-positive rods were rare.
C. Animal treatment: 0.5ml of the gel was intravaginally administered, once a day for consecutive 5 days. The vaginal secretion was sampled on the 6th day. The pH value of vaginal secretion was tested, and the vaginal smear was microscopically examined after Gram-staining. The results were compared with those before treatment.

3. Experimental result
As shown in table 27:

**Table 27 Effects of antimicrobial gel on vaginal flora of rhesus macaque**

| Animal No. | Before administration | | 1 Day after the end of administration | |
|---|---|---|---|---|
| | The vaginal secretion pH | The morphology and quantity of bacteria | The vaginal secretion pH | The morphology and quantity of bacteria |
| 13524 | 5.4 | G^{-b}+++, G^{+c}++, G^{+b} which are large and occasionally observed | 4.8 | G^{+b}, G^{+c}, smaller G^{+c}, WBC++ mostly existed in epithelial cells |

4. Conclusion

The administration of the gel of the present disclosure, which comprised 4.0 percent (weight/volume) of mannose, 0.10 percent (weight/volume) of 2-phenylethanol, and 0.35 percent (weight/volume) of sodium propionate, could partially restore vaginal flora of rhesus macaque toward normal. The vaginal secretion pH value of the rhesus macaque could also be reduced. But the pH value did not yet drop to 4.1 or below.

### Experimental example 18

1. Experimental objective: to observe the modulating effect of the gel containing 6.5 percent (weight/volume) of isomaltooligosaccharide, 0.10 percent (weight/volume) of 2-phenylethanol, and 0.475 percent (weight/volume) of sodium propionate on the vaginal flora of rhesus macaques.
2. Experimental method:
A. Preparation of gel: according to the preparation method of the present disclosure, the gel was prepared by using 6.5 percent (weight/volume) of isomaltooligosaccharide, 0.10 percent (weight/volume) of 2-phenylethanol, 0.475 percent (weight/volume) of sodium propionate, and 2.15 percent (weight/volume) of Xanthan gum. The pH value of the gel was adjusted to 3.3.
B. Animals: two adult female rhesus macaques. The pH values of the vaginal secretion were 5.4. Vaginal smear examination showed dominant Gram-negative rods. Large Gram-positive rods were rare.
C. Animal treatment: 0.5ml of the gel was intravaginally administered, once a day for consecutive 5 days. The vaginal secretion was sampled on the 6th day. The pH values of vaginal secretion were tested, and the vaginal smears were microscopically examined after Gram-staining. The results were compared with those before treatment.

3. Experimental result
As shown in table 28:

**Table 28. Effects of antimicrobial gel on vaginal flora of rhesus macaque**

| Animal No. | Before administration | | 1 Day after the end of administration | |
|---|---|---|---|---|
| | The vaginal secretio n pH | The morphology and quantity of bacteria | The vaginal secretion pH | The morphology and quantity of bacteria |
| 6048 | 5.4 | G^{-b}+++, G^{+c}+, typical BV flora | 3.8-4.1 | G^{-b}++, G^{+b}++, G^{+c}++ |
| 13132 | 5.4 | G^{-b}+++, G^{+c}+~++, G^{+b}+. WBC -, typical BV flora | <3.8 | G^{+b}++ |

4. Conclusion

The administration of the gel of the present disclosure, which comprised 6.5 percent (weight/volume) of isomaltooligosaccharide, 0.10 percent (weight/volume) of 2-phenylethanol, and 0.475 percent (weight/volume) of sodium propionate, could restore the vaginal flora of rhesus macaques to normal. The vaginal secretion pH values of rhesus macaques could also be reduced.

### Experimental example 19

1. Experimental objective: to observe the modulating effect of the gel containing 2.0 percent (weight/volume) of maltose, 3.5 percent (weight/volume) of fructose, 0.10 percent (weight/volume) of 2-phenylethanol, and 0.575 percent (weight/volume) of sodium propionate on the vaginal flora of rhesus macaque,
2. Experimental method:
A. Preparation of gel: according to the preparation method of the present disclosure, the gel was prepared by using 2.0 percent (weight/volume) of maltose, 3.5 percent (weight/volume) of fructose, 0.10 percent(weight/volume) of 2-phenylethanol, 0.575 percent (weight/volume) of sodium propionate, and 2.15 percent (weight/volume) of Xanthan gum. The pH value of the gel was adjusted to 3.3.
B. Animal: one adult female rhesus macaque. The pH value of the vaginal secretion was 5.4. Vaginal smear examination showed dominant Gram-negative rods. Large Gram-positive rods were rare.
C. Animal treatment: 0.5ml of the gel was intravaginally administered, once a day for consecutive 5 days. The vaginal secretion was sampled on the 6th day. The pH value of vaginal secretion was tested, and the vaginal smear was microscopically examined after Gram-staining. The results were compared with those before treatment.

3. Experimental result
As shown in table 29:

**Table 29. Effects of antimicrobial gel on vaginal flora of rhesus macaque**

| Animal No. | Before administration | | 1 Day after the end of administration | |
|---|---|---|---|---|
| | The vaginal secretion pH | The morphology and quantity of bacteria | The vaginal secretion pH | The morphology and quantity of bacteria |
| 13362 | 5.4 | G^{-b}+++, G^{+c}+, G^{+b} occasionally observed | 3.8-4.1 | G^{+b}+++, WBC+ , mostly existed in epithelial cells |

4. Conclusion

The administration of the gel of the present disclosure, which comprised 2.0 percent (weight/volume) of maltose, 3.5 percent (weight/volume) of fructose, 0.10 percent (weight/volume) of 2-phenylethanol, and 0.575 percent (weight/volume) of sodium propionate, could restore vaginal flora of rhesus macaques to normal. The vaginal secretion pH value of rhesus macaque could also be reduced.

### Experimental example 21

1. Experimental objective: to observe the modulating effect of the gel containing 3.5 percent (weight/volume) of sucrose, 1.0 percent (weight/volume) of maltose, 0.06 percent (weight/volume) of 2-phenylethanol, 0.525 percent (weight/volume) of sodium propionate, and 0.15 percent (weight/volume) of sodium benzoate on the vaginal flora of rhesus macaque.
2. Experimental method:
A. Preparation of gel: according to the preparation method of the present disclosure, the gel was prepared by using 3.5 percent (weight/volume) of sucrose, 1.0 percent (weight/volume) of maltose, 0.06 percent (weight/volume) of 2-phenylethanol, 0.525 percent (weight/volume) of sodium propionate, 0.15 percent (weight/volume) of sodium benzoate, and 2.15 percent (weight/volume) of Xanthan gum. The pH value of the gel was adjusted to 3.3.
B. Animals: two adult female rhesus macaques. The pH values of the vaginal secretion were all 5.4. The vaginal smear examination showed dominant Gram-negative rods. Large Gram-positive rods were rare.
C. Animal treatment: 0.5ml of the gel was intravaginally administered, once a day for consecutive 5 days. The vaginal secretion was sampled with a swab on the 6th day. The pH values of vaginal secretion were tested, and the vaginal smears were microscopically examined after Gram-staining. The results were compared with those before treatment.

3. Experimental result
As shown in table 31:

**Table 31. Effects of antimicrobial gel on vaginal flora of rhesus macaque**

| Animal No. | Before administration | | 1 Day after the end of administration | |
|---|---|---|---|---|
| | The vaginal secretio n pH | The morphology and quantity of bacteria | The vaginal secretion pH | The morphology and quantity of bacteria |
| 3902 | 5.4 | G^{-b}+++, G^{+c}+, typical BV flora | 4.1 | G^{-b} ++, G^{+b}+++ |
| 13060-2 | 5.4 | G^{-b}+++, G^{+b} occasionally observed | 5.4 | G^{-b} +, WBC++ |

4. Conclusion

After vaginal administration of the gel of the present disclosure, which comprised 3.5 percent (weight/volume) of sucrose, 1.0 percent (weight/volume) of maltose, 0.06 percent (weight/volume) of 2-phenylethanol, 0.525 percent (weight/volume) of sodium propionate, and 0.15 percent (weight/volume) of sodium benzoate, the pH value of vaginal secretion of one rhesus macaque decreased from 5.4 to 4.1, while G^{-b} significantly decreased and G^{+b} significantly increased. The vaginal flora basically restored to normal. Although the pH value of vaginal secretion of the other one rhesus macaque did not change, the amount of G^{-b} bacteria in vaginal reduced. It was suggested that the composition was effective in modulating vaginal flora and could partially restore the vaginal flora of rhesus macaque as well as the vaginal pH values to normal.

### Experimental example 22

1. Experimental objective: to observe the modulating effect of the gel containing 3.0 percent (weight/volume) of maltose, 1.0 percent (weight/volume) of fructose, 0.065 percent (weight/volume) of 2-phenylethanol, 0.55 percent (weight/volume) of sodium propionate, and 0.12 percent (weight/volume) of sodium benzoate on the vaginal flora of rhesus macaques
2. Experimental method:
A. Preparation of gel: according to the preparation method of the present disclosure, the gel was prepared by using 3.0 percent (weight/volume) of maltose, 1.0 percent (weight/volume) of fructose, 0.065 percent (weight/volume) of 2-phenylethanol, 0.55 percent (weight/volume) of sodium propionate, 0.12 percent (weight/volume) of sodium benzoate, and 2.15 percent (weight/volume) of Xanthan gum. The pH value of the gel was adjusted to 3.3.
B. Animals: eight adult female rhesus macaques. The pH values of the vaginal secretion were all 5.4. The vaginal smear examination showed dominant Gram-negative rods. Large Gram-positive rods were rare.
C. Animal treatment: 0.5ml of the gel was intravaginally administered, once a day for consecutive 5 days. Vaginal secretion was sampled on the 6th day. The pH values of vaginal secretion were tested, and the vaginal smears were microscopically examined after Gram-staining. The results were compared with those before treatment.

3. Experimental result
As shown in table 32:

**Table 32. Effects of antimicrobial gel on vaginal flora of rhesus macaque**

| Animal No. | Before administration | | 1 Day after the end of administration | |
|---|---|---|---|---|
| | The vaginal secretion pH | The morphology and quantity of bacteria | The vaginal secretio n pH | The morphology and quantity of bacteria |
| 13296 | >5.4 | G^{-b}+++, typical BV flora | 5.4 | G^{+b}+++ |
| 13684 | 5.4 | G^{-b}+++, G^{+c}+, WBC+ | <3.8 | G^{-b}+, G^{+b}+++ |
| 3774 | 5.4 | G^{-b}+++, G^{+c}+, typical BV flora | <3.8 | G^{-b}+, G^{+b}+++ |
| 13442 | 5.4 | G^{-b}+++, G^{+c}+, typical BV flora | <3.8 | G^{+b}+++, G^{+c}+ |
| 11290 | 5.4 | G^{-b}+++, typical BV flora | 4.1 | G^{-b}+, G^{+b}+++, WBC+ |
| 13588 | 5.4 | G^{-b}+++, typical BV flora | <3.8 | G^{+b}+++ |
| 13468 | 5.4 | G^{-b}+++, typical BV flora | 4.1 | G^{-b}+, G^{+b}+++, WBC+ |
| 4086 | 5.4 | G^{-b}+++, typical BV flora | 5.4 | G^{-b}+++, G^{+c}+++ |

4. Conclusion

The gel of the present disclosure, which comprised 3.0 percent (weight/volume) of maltose, 1.0 percent (weight/volume) of fructose, 0.065 percent (weight/volume) of 2-phenylethanol, 0.55 percent (weight/volume) of sodium propionate, and 0.12 percent (weight/volume) of sodium benzoate, were applied to eight rhesus macaques. The pH values of vaginal secretion of four rhesus macaques were all decreased from 5.4 to below 3.8, while the vaginal flora restored to normal. The pH values of vaginal secretion of two rhesus macaques were decreased from 5.4 to 4.1, while the vaginal flora were dominated by large Gram-positive rods. The pH values of vaginal secretion of two rhesus macaques did not change, wherein the vaginal flora of one restored normal and the other one remained BV-typ. It was shown that the gel was effective in modulating the vaginal flora of rhesus macaques, restoring the vaginal flora, and decreasing the pH values of vaginal secretion.

### Experimental example 23

1. Experimental objective: to observe the modulating effect of the gel containing 0.10 percent (weight/volume) of 2-phenylethanol, 0.575 percent (weight/volume) of sodium propionate, and 0.09 percent (weight/volume) of sodium benzoate on the vaginal flora of rhesus macaque.
2. Experimental method:
A. Preparation of gel: according to the preparation method of the present disclosure, the gel was prepared by using 0.10 percent (weight/volume) of 2-phenylethanol, 0.575 percent (weight/volume) of sodium propionate, 0.09 percent (weight/volume) of sodium benzoate, and 2.15 percent (weight/volume) of Xanthan gum. The pH value of the gel was adjusted to 3.3.
B. Animals: four adult female rhesus macaques. The pH values of the vaginal secretion were all 5.4. The vaginal smear examination showed dominant Gram-negative rods. Large Gram-positive rods were rare.
C. Animal treatment: 0.5ml of the gel was intravaginally administered, once a day for consecutive 5 days. The vaginal secretion was sampled on the 6th day. The pH values of vaginal secretion were tested, and the vaginal smears were microscopically examined after Gram-staining. The results were compared with those before treatment.

3. Experimental result
As shown in table 33:

**Table 33. Effects of antimicrobial gel on vaginal flora of rhesus macaque**

| Animal No. | Before administration | | 1 Day after the end of administration | |
|---|---|---|---|---|
| | The vaginal secretion pH | The morphology and quantity of bacteria | The vaginal secretion pH | The morphology and quantity of bacteria |
| 13174 | 5.4 | G^{-b}+++, typical BV flora | 5.4 | G^{+b}+, WBC+ |
| 13126 | 5.4 | G^{-b}+++, WBC+ | 4.1 | G^{+b}+++, WBC+ |
| 13508 | 5.4 | G^{-b}+++, G^{+c}+, G^{+b} occasionally observed | 5.4 | G^{+b} occasionally observed, G^{-b}+, WBC+ |
| 6924 | 5.4 | G^{-b}+++, WBC+ | 5.4 | G^{+b}+, WBC+ |

4. Conclusion

After vagianl administration of the gel of the present disclosure, which comprised 0.10 percent (weight/volume) of 2-phenylethanol, 0.575 percent (weight/volume) of sodium propionate, and 0.09 percent (weight/volume) of sodium benzoate, the pH value of vaginal secretion of one rhesus macaque decreased from 5.4 to 4.1, while the vaginal flora became dominated by Gram-positive bacilli. The pH values of vaginal secretion of the other three rhesus macaques did not decrease. It was suggested that the gel could modulate the vaginal flora of rhesus macaque. But the effect in restoring the vaginal flora and decreasing the vaginal pH value were not as good as those of the saccharide-containing preparation of the present disclosure.

### Experimental example 24

1. Experimental objective: to observe the modulating effect of the gel containing 0.60 percent (weight/volume) of 2-phenylethanol and 0.70 percent (weight/volume) of sodium propionate on the vaginal flora of rhesus macaque.
2. Experimental method:
A. Preparation of gel: according to the preparation method of the present disclosure, the gel was prepared by using 0.60 percent (weight/volume) of 2-phenylethanol, 0.70 percent (weight/volume) of sodium propionate, and 2.15 percent (weight/volume) of Xanthan gum. The pH value of the gel was adjusted to 4.1.
B. Animals: two adult female rhesus macaques. The pH values of the vaginal secretion were both 5.4. The vaginal smear examination showed dominant Gram-negative rods. Large Gram-positive rods were rare.
C. Animal treatment: 0.5ml of the gel was intravaginally administered, once a day for consecutive 5 days. The vaginal secretion was sampled on the 6th day. The pH values of vaginal secretion were tested, and the vaginal smear were microscopically examined after Gram-staining. The results were compared with those before treatment.

3. Experimental result
As shown in table 34:

**Table 34. Effects of antimicrobial gel on vaginal flora of rhesus macaque**

| Animal No. | Before administration | | 1 Day after the end of administration | |
|---|---|---|---|---|
| | The vaginal secretio n pH | The morphology and quantity of bacteria | The vaginal secretion pH | The morphology and quantity of bacteria |
| 13262 | 5.4 | G^{-b}+++, typical BV flora | 5.4 | Few bacteria, WBC+ |
| 13187 | 5.4 | G^{-b}+++, WBC++ | 5.4 | Few bacteria, G^{+b}+ occasionally observed, WBC+ |

4. Conclusion

After administering the gel of the present disclosure, which comprised 0.60 percent (weight/volume) of 2-phenylethanol and 0.70 percent (weight/volume) of sodium propionate, the number of vaginal bacteria in two rhesus macaques were significantly reduced, while large Gram-positive bacilli (G^{+b}) were occasionally observed in one rhesus macaques. However, the pH values of vaginal secretion of both rhesus macaques were not reduced. It was suggested that the gel of the present disclosure containing "0.60 percent (weight/volume) of 2-phenylethanol and 0.70 percent (weight/volume) of sodium propionate" could inhibit vaginal bacteria, especially Gram-negative bacilli (G^{-b}). Although large Gram-positive bacilli (G^{+b}) were occasionally observed in vaginal secretion of one rhesus macaque, the flora did not restore to normal, and the pH values of vaginal secretion did not decrease to 4.1 or below.

### Experimental example 25

1. Experimental objective: to observe the modulating effect of the gel containing 2.0 percent (weight/volume) of fructose, 4.0 percent (weight/volume) of isomaltose, 0.06 percent (weight/volume) of 2-phenylethanol, 0.575 percent (weight/volume) of sodium propionate, and 0.12 percent (weight/volume) of sodium benzoate on the vaginal flora of rhesus macaque.
2. Experimental method:
A. Preparation of gel: according to the preparation method of the present disclosure, the gel was prepared by using 2.0 percent (weight/volume) of fructose, 4.0 percent (weight/volume) of isomaltose, 0.06 percent (weight/volume) of 2-phenylethanol, 0.575 percent (weight/volume) of sodium propionate, 0.12 percent (weight/volume) of sodium benzoate, and 2.15 percent (weight/volume) of Xanthan gum The pH value of the gel was adjusted to 3.3.
B. Animals: three adult female rhesus macaques. All the pH values of the vaginal secretion were 5.4. Vaginal smear examination showed dominant Gram-negative rods. Large Gram-positive rods were rare.
C. Animal treatment: 0.5ml of the gel was intravaginally administered, once a day for consecutive 5 days. The vaginal secretion was sampled with swabs on the 6th day. The pH values of vaginal secretion were tested, and the vaginal smears were microscopically examined after Gram-staining. The results were compared with those before treatment.

3. Experimental result
As shown in table 35:

**Table 30. Effects of antimicrobial gel on vaginal flora of rhesus macaque**

| Animal No. | Before administration | | 1 Day after the end of administration | |
|---|---|---|---|---|
| | The vaginal secretio n pH | The morphology and quantity of bacteria | The vaginal secretio n pH | The morphology and quantity of bacteria |
| 12266 | 5.4 | G^{-b}+++, typical BV flora | <3.8 | G^{+b}+, WBC+ |
| 3902 | 5.4 | G^{-b}+++, WBC+ | <3.8 | G^{+b}+++, WBC+ |
| 3790 | 5.4 | G^{-b}+++, G^{+c}+, G^{+b} occasionally observed | 4.1 | G^{+b} occasionally observed, G^{-b}+, WBC+ |

4. Conclusion

The administration of the gel of the present disclosure, which comprised 2.0 percent (weight/volume) of fructose, 4.0 percent (weight/volume) of isomaltose, 0.06 percent (weight/volume) of 2-phenylethanol, 0.575 percent (weight/volume) of sodium propionate, and 0.12 percent (weight/volume) of sodium benzoate, could restore the vaginal flora of rhesus macaques to normal. The vaginal secretion pH values of rhesus macaques could also be reduced.

### Experimental example 26

1. Experimental objective: to observe the modulating effect of the gel containing 5.5 percent (weight/volume) of maltose, 1.0 percent (weight/volume) of isomaltulose, 0.10 percent(weight/volume) of 2-phenylethanol, 0.575 percent (weight/volume) of sodium propionate, and 0.065 percent (weight/volume) of sodium benzoate on the vaginal flora of rhesus macaque.
2. Experimental method:
A. Preparation of gel: according to the preparation method of the present disclosure, the gel was prepared by using 5.5 percent (weight/volume) of maltose, 1.0 percent (weight/volume) of isomaltulose, 0.10 percent (weight/volume) of 2-phenylethanol, 0.575 percent (weight/volume) of sodium propionate, 0.065 percent (weight/volume) of sodium benzoate, and 2.15 percent (weight/volume) of Xanthan gum. The pH value of the gel was adjusted to 4.3.
B. Animals: two adult female rhesus macaques. The pH values of the vaginal secretion were 5.4. Vaginal smear examination showed dominant Gram-negative rods. Large Gram-positive rods were rare.
C. Animal treatment: 0.5ml of the gel was intravaginally administered, once a day for consecutive 5 days. The vaginal secretion was sampled with swabs on the 6th day. The pH values of vaginal secretion were tested, and the vaginal smears were microscopically examined after Gram-staining. The results were compared with those before treatment.

3. Experimental result
As shown in table 36:

**Table 36. Effects of antimicrobial gel on vaginal flora of rhesus macaque**

| Animal No. | Before administration | | 1 Day after the end of administration | |
|---|---|---|---|---|
| | The vaginal secretio n pH | The morphology and quantity of bacteria | The vaginal secretion pH | The morphology and quantity of bacteria |
| 13444 | 5.4 | G^{-b}++++, G^{+c}+, typical BV flora | <3.8 | G^{+b}++, WBC+-++ |
| 13126 | 5.4 | G^{-b}++++, G^{+c}++, G^{+b}+ occasionally observed, typical BV flora, WBC+ | 4.1 | G^{+b}++ |

4. Conclusion

The administration of the gel of the present disclosure, which comprised 5.5 percent (weight/volume) of maltose, 1.0 percent (weight/volume) of isomaltulose, 0.10 percent (weight/volume) of 2-phenylethanol, 0.575 percent (weight/volume) of sodium propionate, and 0.065 percent (weight/volume) of sodium benzoate, could restore the vaginal flora of rhesus macaques to normal. The vaginal secretion pH values of rhesus macaques could also be reduced.

### Experimental example 27

1. Experimental objective: to observe the modulating effect of the gel containing 2.0 percent (weight/volume) of maltose, 0.20 percent (weight/volume) of 2-phenylethanol, 0.575 percent (weight/volume) of sodium propionate, and 0.065 percent (weight/volume) of sodium benzoate on the vaginal flora of rhesus macaque.
   A. Preparation of gel: according to the preparation method of the present disclosure, the gel was prepared by using 2.0 percent (weight/volume) of maltose, 0.20 percent (weight/volume) of 2-phenylethanol, 0.575 percent (weight/volume) of sodium propionate, 0.065 percent (weight/volume) of sodium benzoate, and 2.15 percent (weight/volume) of Xanthan gum. The pH value of the gel was adjusted to 3.3.
   B. Animals: five adult female rhesus macaques. The pH values of the vaginal secretion were all 5.4. The vaginal smear examination showed dominant Gram-negative rods. Large Gram-positive rods were rare.
   C. Animal treatment: 0.5ml of the gel was intravaginally administered, once a day for consecutive 5 days. Vaginal secretion was sampled with swabs on the 6th day. The pH values of vaginal secretion were tested, and the vaginal smears were microscopically examined after Gram-staining. The results were compared with those before treatment.

### 3. Experimental result

As shown in table 37:

**Table 37. Effects of antimicrobial gel on vaginal flora of rhesus macaque**

| Anim al No. | Before administration | | 1 Day after the end of administration | |
|---|---|---|---|---|
| | The vaginal secreti on pH | The morphology and quantity of bacteria | The vaginal secretio n pH | The morphology and quantity of bacteria |
| 6374 | 5.4 | G^{-b}++++, G^{+c}+, typical BV flora | 4.1 | G^{+b}+++ |
| 1329 6 | 5.4 | G^{-b}++++, G^{+c}++, G^{+b}+ occasionally observed, typical BV flora, WBC+ | 4.1 | G^{+b}+++ |
| 1351 8 | 5.4 | G^{-b}++++, G^{+c}+, typical BV flora | 4.1 | G^{+b}+++ |
| 1128 4 | 5.4 | G^{-b}++++, G^{+c}+, typical BV flora | 4.4 | G^{+b}+++, G^{+c}+ |

### 4. Conclusion

The administration of the gel of the present disclosure, which comprised 2.0 percent (weight/volume) of maltose, 0.20 percent (weight/volume) of 2-phenylethanol, 0.575 percent (weight/volume) of sodium propionate, and 0.065 percent (weight/volume) of sodium benzoate, could restore the vaginal flora of rhesus macaques to normal. The vaginal secretion pH values of rhesus macaques could also be reduced.

## Claims

1. A composition for vagina for use in promoting the restoration of beneficial Lactobacilli, increasing vaginal acidity, eliminating and/or alleviating vaginal discomforts comprising vulvovaginal pruritus or pain, and/or sexual intercourse pain, improving leucorrhea properties, eliminating leucorrhea smell, and/or preventing and/or treating vaginal infectious diseases comprising cytolytic vaginosis, vaginal dysbacteriosis, atrophic vaginitis, aerobic vaginitis, and/or bacterial vaginosis, wherein the composition comprises the following antimicrobial agents: (1) 2-phenylethanol, the total content of which is 0.05 to 0.60 percent (weight/volume); (2) propionic acid and/or sodium propionate and/or calcium propionate, the total content of which, calculated as sodium propionate, is 0.25 to 0.70 percent (weight/volume), and/or benzoic acid and/or sodium benzoate, the total content of which, calculated as sodium benzoate, is 0.05 to 0.15 percent (weight/volume) ; wherein the pH value of the composition is from 3.0 to 4.3, and the dosage form of the composition is a solution, a water-soluble gel, or an emulsive ointment.

2. The composition for vagina according to claim 1 for use in promoting the restoration of beneficial Lactobacilli, increasing vaginal acidity, eliminating and/or alleviating vaginal discomforts comprising vulvovaginal pruritus or pain, and/or sexual intercourse pain, improving leucorrhea properties, eliminating leucorrhea smell, and/or preventing and/or treating vaginal infectious diseases comprising cytolytic vaginosis, vaginal dysbacteriosis, atrophic vaginitis , aerobic vaginitis, and/or bacterial vaginosis, wherein the total content of 2-phenylethanol is from 0.10 to 0.40 percent (weight/volume); the total content of propionic acid and/or sodium propionate and/or calcium propionate, calculated as sodium propionate, is from 0.525 to 0.575 percent (weight/volume); the total content of benzoic acid and/or sodium benzoate, calculated as sodium benzoate, is from 0.065 to 0.090 percent (weight/volume).

3. The composition for vagina according to claim 1 for use in promoting the restoration of beneficial Lactobacilli, increasing vaginal acidity, eliminating and/or alleviating vaginal discomforts comprising vulvovaginal pruritus or pain, and/or sexual intercourse pain, improving leucorrhea properties, eliminating leucorrhea smell, and/or preventing and/or treating vaginal infectious diseases comprising cytolytic vaginosis, vaginal dysbacteriosis, atrophic vaginitis, aerobic vaginitis, and/or bacterial vaginosis, wherein the antimicrobial agents are 2-phenylethanol, propionic acid and/or sodium propionate, and benzoic acid and/or sodium benzoate.

4. The composition for vagina according to claim 1 for use in promoting the restoration of beneficial Lactobacilli, increasing vaginal acidity, eliminating and/or alleviating vaginal discomforts comprising vulvovaginal pruritus or pain, and/or sexual intercourse pain, improving leucorrhea properties, eliminating leucorrhea smell, and/or preventing and/or treating vaginal infectious diseases comprising cytolytic vaginosis, vaginal dysbacteriosis, atrophic vaginitis, aerobic vaginitis, and/or bacterial vaginosis, wherein the composition for vagina further comprises one or more antimicrobial agents selected from a group consisting of dehydroacetic acid, sodium dehydroacetate, sorbic acid, potassium sorbate, sodium sorbate, diacetic acid, sodium diacetate, caprylic acid, sodium caprylate, capric acid, sodium caprate, undecylenic acid, sodium undecylenate, lauric acid, sodium laurate, natamycin, lactoferrin, lactoferrin peptide, lysozyme, antibacterial protein, antibacterial peptide, lichenic acid, bergenin, tropolone, chlorogenic acid, palmatine, benzyl alcohol, propylene phenoxyethanol, 1,2-pentanediol, 1,2-hexanediol, 1,6-hexanediol, 1,2-octanediol, 1,2-decanediol, 2-methyl-1,3-propanediol, ethylhexylglycerin, and benzoyl peroxide.

5. A composition for vagina, wherein the composition comprises: (1) 2-phenylethanol, the total content of which is 0.05 to 0.60 percent (weight/volume); (2) propionic acid and/or sodium propionate and/or calcium propionate, the total content of which, calculated as sodium propionate, is 0.25 to 0.70 percent (weight/volume), and/or benzoic acid and/or sodium benzoate, the total content of which, calculated as sodium benzoate, is 0.05 to 0.15 percent (weight/volume);
wherein, the dosage form of the composition is a solution, a water-soluble gel, or an emulsive ointment, in which the pH value of the composition is from 3.0 to 4.3.

6. The composition according to claim 5, wherein the total content of 2-phenylethanol is from 0.10 to 0.40 percent (weight/volume); the total content of propionic acid and/or sodium propionate and/or calcium propionate, calculated as sodium propionate, is from 0.525 to 0.575 percent (weight/volume); the total content of benzoic acid and/or sodium benzoate, calculated as sodium benzoate, is from 0.065 to 0.090 percent (weight/volume).

7. The composition according to claim 5, wherein the antimicrobial agents are 2-phenylethanol, propionic acid and/or sodium propionate, and benzoic acid and/or sodium benzoate.

8. The composition according to claim 5, wherein the composition further comprises one or more antimicrobial agents selected from a group consisting of dehydroacetic acid, sodium dehydroacetate, sorbic acid, potassium sorbate, sodium sorbate, diacetic acid, sodium diacetate, caprylic acid, sodium caprylate, capric acid, sodium caprate, undecylenic acid, sodium undecylenate, lauric acid, sodium laurate, natamycin, lactoferrin, lactoferrin peptide, lysozyme, antibacterial protein, antibacterial peptide, lichenic acid, bergenin, tropolone, chlorogenic acid, palmatine, benzyl alcohol, propylene phenoxyethanol, 1,2-pentanediol, 1,2-hexanediol, 1,6-hexanediol, 1,2-octanediol, 1,2-decanediol, 2-methyl-1, 3-propanediol, ethylhexylglycerin, and benzoyl peroxide.

9. The composition according to claim 5, wherein the composition further comprises one or more estrogens and/or phytoestrogens agents selected from a group consisting of diethylstilbestrol, estradiol, estriol, daidzein, aglycone of daidzein, genistin, glycitein, aglycone of glycitein, biochanin, coumestrol, and formononetin, with the total content of estrogens and/or phytoestrogens agents ranging from 0.001 to 1.0 percent (weight/volume).

10. The composition according to claim 5, wherein the composition further comprises one or more saccharides selected from a group consisting of glucose, fructose, mannose, sucrose, isomaltulose, 1-kestose, nistose trihydrate, 1F-fructofuranosylnystose, maltose, isomaltose, isomaltotriose, isomaltotetraose, isomaltopentaose, trehalose, cellobiose, melibiose, gentiobiose, gentiooligosaccharide, raffinose, panose, maltooligosaccharide, isomaltulooligosaccharide, fructooligosaccharide, glucomannan, dextrin, starch, and glycogen, with the total content of saccharides ranging from 1.0 to 9.0 percent (weight/volume).

11. The composition according to claim 10, wherein the saccharides are selected from a group consisting of glucose, fructose, mannose, sucrose, maltose, isomaltulose, isomaltose, trehalose, and maltooligosaccharide, with the total content of 2.0 percent to 6.5 percent (weight/volume).

12. The composition according to claim 5, wherein the composition further comprises one or more substances selected from a group consisting of 0.01 to 5.0 percent (weight/volume) of aloe extract, 0.01 to 5.0 percent (weight/volume) of lavender extract, 0.001 to 1.0 percent (weight/volume) of vitamin E, 0.001 to 1.0 percent (weight/volume) of vitamin A, 0.001 to 1.0 percent (weight/volume) of vitamin D, and 0.001 to 1.0 percent of (weight/volume) vitamin C.

13. The composition according to claim 5, wherein the composition further comprises one or more amino acids and/or physiologically acceptable salts, which is selected from a group consisting of glutamic acid, glutamine, aspartic acid, asparagine, isoleucine, phenylalanine, valine, leucine, proline, and threonine, with the total content of amino acids ranging from 0.1 to 10.0 percent (weight/volume).

14. The composition according to claim 13, wherein the content of the amino acid is 1.0 to 5.0 percent (weight/volume), and the amino acid is glutamic acid and/or aspartic acid.

15. A preparation method of a composition for vagina, wherein the method comprises the operation of adding the following agents into water, and/or water-soluble gel matrix, and/or emulsive ointment matrix: (1) 2-phenylethanol, the total content of which is 0.05 to 0.60 percent (weight/volume); (2) propionic acid and/or sodium propionate and/or calcium propionate, the total content of which, calculated as sodium propionate, is 0.25 to 0.70 percent (weight/volume), and/or benzoic acid and/or sodium benzoate, the total content of which, calculated as sodium benzoate, is 0.05 to 0.15 percent (weight/volume); wherein the pH value of the composition is from 3.0 to 4.3, and the dosage form of the composition is a solution, a water-soluble gel, or an emulsive ointment.

## Patentansprüche

1. Eine Zusammensetzung für die Vagina zur Förderung der Wiederherstellung von nützlichen Laktobazillen, zur Erhöhung des vaginalen Säuregehalts, zur Beseitigung und/oder Linderung von vaginalen Beschwerden, einschließlich vulvovaginalem Juckreiz oder Schmerzen, und/oder Schmerzen beim Geschlechtsverkehr, zur Verbesserung der Eigenschaften von Leukorrhoe, zur Beseitigung des Leukorrhoe-Geruchs und/oder Verhinderung und/oder Behandlung vaginaler Infektionskrankheiten umfassend die zytolytische Vaginose, vaginale Dysbakteriose, atrophische Vaginitis, aerobe Vaginitis und/oder bakterielle Vaginose, wobei die Zusammensetzung die folgenden antimikrobiellen Wirkstoffe umfasst: (1) 2-Phenylethanol, dessen Gesamtgehalt 0,05 bis 0,60 Prozent (Gewicht/Volumen) beträgt; (2) Propionsäure und/oder Natriumpropionat und/oder Calciumpropionat, deren Gesamtgehalt, berechnet als Natriumpropionat, 0,25 bis 0,70 Prozent (Gewicht/Volumen) beträgt, und/oder Benzoesäure und/oder Natriumbenzoat, deren Gesamtgehalt, berechnet als Natriumbenzoat, 0,05 bis 0,15 Prozent (Gewicht/Volumen) beträgt; wobei der pH-Wert der Zusammensetzung 3,0 bis 4,3 beträgt und die Darreichungsform der Zusammensetzung eine Lösung, ein wasserlösliches Gel oder eine emulgierende Salbe ist.

2. Die Zusammensetzung für die Vagina gemäß Anspruch 1 zur Verwendung bei der Förderung der Wiederherstellung von nützlichen Laktobazillen, zur Erhöhung des vaginalen Säuregehalts, zur Beseitigung und/oder Linderung von vaginalen Beschwerden, einschließlich vulvovaginalem Juckreiz oder Schmerzen, und/oder Schmerzen beim Geschlechtsverkehr, zur Verbesserung der Eigenschaften von Leukorrhoe, zur Beseitigung des Leukorrhoe-Geruchs und/oder Verhinderung und/oder Behandlung vaginaler Infektionskrankheiten umfassend die zytolytische Vaginose, vaginale Dysbakteriose, atrophische Vaginitis, aerobe Vaginitis und/oder bakterielle Vaginose, wobei der Gesamtgehalt an 2-Phenylethanol 0,10 bis 0,40 Prozent (Gewicht/Volumen) beträgt; der Gesamtgehalt an Propionsäure und/oder Natriumpropionat und/ oder Calciumpropionat, berechnet als Natriumpropionat, 0,525 bis 0,575 Prozent (Gewicht/Volumen) beträgt; der Gesamtgehalt an Benzoesäure und/oder Natriumbenzoat, berechnet als Natriumbenzoat, 0,065 bis 0,090 Prozent (Gewicht/Volumen) beträgt.

3. Die Zusammensetzung für die Vagina gemäß Anspruch 1 zur Verwendung bei der Förderung der Wiederherstellung von nützlichen Laktobazillen, zur Erhöhung des vaginalen Säuregehalts, zur Beseitigung und/oder Linderung von vaginalen Beschwerden, einschließlich vulvovaginalem Juckreiz oder Schmerzen, und/oder Schmerzen beim Geschlechtsverkehr, zur Verbesserung der Eigenschaften von Leukorrhoe, zur Beseitigung des Leukorrhoe-Geruchs und/oder Verhinderung und/oder Behandlung vaginaler Infektionskrankheiten umfassend die zytolytische Vaginose, vaginale Dysbakteriose, atrophische Vaginitis, aerobe Vaginitis und/oder bakterielle Vaginose, wobei die antimikrobiellen Wirkstoffe 2-Phenylethanol, Propionsäure und/oder Natriumpropionat und Benzoesäure und/oder Natriumbenzoat sind.

4. Die Zusammensetzung für die Vagina gemäß Anspruch 1 zur Verwendung bei der Förderung der Wiederherstellung von nützlichen Laktobazillen, zur Erhöhung des vaginalen Säuregehalts, zur Beseitigung und/oder Linderung von vaginalen Beschwerden, einschließlich vulvovaginalem Juckreiz oder Schmerzen, und/oder Schmerzen beim Geschlechtsverkehr, zur Verbesserung der Eigenschaften von Leukorrhoe, zur Beseitigung des Leukorrhoe-Geruchs und/oder Verhinderung und/oder Behandlung vaginaler Infektionskrankheiten umfassend die zytolytische Vaginose, vaginale Dysbakteriose, atrophische Vaginitis, aerobe Vaginitis und/oder bakterielle Vaginose, wobei die Zusammensetzung für die Vagina ferner ein oder mehrere antimikrobielle Mittel umfasst, die aus einer Gruppe ausgewählt sind, umfassend Dehydroessigsäure, Natriumdehydroacetat, Sorbinsäure, Kaliumsorbat, Natriumsorbat, Diessigsäure, Natriumdiacetat, Caprylsäure , Natriumcaprylat, Caprinsäure, Natriumcaprat, Undecylensäure, Natriumundecylenat, Laurinsäure, Natriumlaurat, Natamycin, Lactoferrin, Lactoferrinpeptid, Lysozym, antibakterielles Protein, antibakterielles Peptid, Licheninsäure, Bergenin, Tropolon, Chlorogensäure, Palmatin, Benzylalkohol, Propylenphenoxyethanol, 1,2-Pentandiol, 1,2-Hexandiol, 1,6-Hexandiol, 1,2-Octandiol, 1,2-Decandiol, 2-Methyl-1,3-propandiol, Ethylhexylglycerin und Benzoylperoxid.

5. Zusammensetzung für die Vagina, wobei die Zusammensetzung umfasst: (1) 2-Phenylethanol, dessen Gesamtgehalt 0,05 bis 0,60 Prozent (Gewicht/Volumen) beträgt; (2) Propionsäure und/oder Natriumpropionat und/oder Calciumpropionat, deren Gesamtgehalt, berechnet als Natriumpropionat 0,25 bis 0,70 Prozent (Gewicht/Volumen) beträgt, und/oder Benzoesäure und/oder Natriumbenzoat, deren Gesamtgehalt, berechnet als Natriumbenzoat, 0,05 bis 0,15 Prozent (Gewicht/Volumen) beträgt;
wobei die Dosierungsform der Zusammensetzung eine Lösung, ein wasserlösliches Gel oder eine emulgierende Salbe ist, in der der pH-Wert der Zusammensetzung 3,0 bis 4,3 beträgt.

6. Zusammensetzung nach Anspruch 5, wobei der Gesamtgehalt an 2-Phenylethanol 0,10 bis 0,40 Prozent (Gewicht/Volumen) beträgt; der Gesamtgehalt an Propionsäure und/oder Natriumpropionat und/oder Calciumpropionat, berechnet als Natriumpropionat, 0,525 bis 0,575 Prozent (Gewicht/Volumen) beträgt; der Gesamtgehalt an Benzoesäure und/oder Natriumbenzoat, berechnet als Natriumbenzoat, 0,065 bis 0,090 Prozent (Gewicht/Volumen) beträgt.

7. Zusammensetzung gemäß Anspruch 5, wobei die antimikrobiellen Mittel 2-Phenylethanol, Propionsäure und/oder Natriumpropionat und Benzoesäure und/oder Natriumbenzoat sind.

8. Zusammensetzung nach Anspruch 5, wobei die Zusammensetzung ferner ein oder mehrere antimikrobielle Mittel umfasst, die aus einer Gruppe ausgewählt sind, umfassend Dehydroessigsäure, Natriumdehydroacetat, Sorbinsäure, Kaliumsorbat, Natriumsorbat, Diessigsäure, Natriumdiacetat, Caprylsäure, Natriumcaprylat, Caprinsäure, Natriumcaprat , Undecylensäure, Natriumundecylenat, Laurinsäure, Natriumlaurat, Natamycin, Lactoferrin, Lactoferrinpeptid, Lysozym, antibakterielles Protein, antibakterielles Peptid, Licheninsäure, Bergenin, Tropolon, Chlorogensäure, Palmatin, Benzylalkohol, Propylenphenoxyethanol , 1,2-Pentandiol, 1,2-Hexandiol, 1,6-Hexandiol, 1,2-Octandiol, 1,2-Decandiol, 2-Methyl-1,3-propandiol, Ethylhexylglycerin und Benzoylperoxid.

9. Zusammensetzung nach Anspruch 5, wobei die Zusammensetzung ferner ein oder mehrere östrogene und/oder phytoöstrogene Mittel umfasst, die aus einer Gruppe ausgewählt sind, umfassend Diethylstilböstrol, Estradiol, Östriol, Daidzein, Aglykon von Daidzein, Genistin , Glycitein, Aglycon von Glycitein, Biochanin, Coumestrol und Formononetin, wobei der Gesamtgehalt an östrogenen und/oder phytoöstrogenen Wirkstoffen im Bereich von 0,001 bis 1,0 Prozent (Gewicht/Volumen) liegt.

10. Zusammensetzung nach Anspruch 5, wobei die Zusammensetzung ferner ein oder mehrere Saccharide umfasst, die aus einer Gruppe ausgewählt sind, umfassend Glucose, Fructose, Mannose, Saccharose, Isomaltulose, 1-Kestose, Nistose-Trihydrat, 1F-Fructofuranosylnystose, Maltose, Isomaltose, Isomaltotriose, Isomaltotetraose, Isomaltopentaose, Trehalose, Cellobiose, Melibiose, Gentiobiose, Gentiooligosaccharid, Raffinose, Panose, Maltooligosaccharid, Isomaltulooligosaccharid, Fructooligosaccharid, Glucomannan, Dextrin, Stärke und Glycogen, wobei der Gesamtgehalt an Sacchariden im Bereich von 1,0 bis 9,0 Prozent (Gewicht/Volumen) liegt.

11. Zusammensetzung nach Anspruch 10, wobei die Saccharide aus einer Gruppe ausgewählt sind, umfassend Glucose, Fructose, Mannose, Saccharose, Maltose, Isomaltulose, Isomaltose, Trehalose und Maltooligosaccharid, mit einem Gesamtgehalt von 2,0 Prozent bis 6,5 Prozent (Gewicht/Volumen).

12. Zusammensetzung nach Anspruch 5, wobei die Zusammensetzung ferner eine oder mehrere Substanzen umfasst, die aus einer Gruppe ausgewählt sind, umfassend 0,01 bis 5,0 Prozent (Gewicht/Volumen) Aloe-Extrakt, 0,01 bis 5,0 Prozent (Gewicht/Volumen) Lavendel-Extrakt, 0,001 bis 1, 0 Prozent (Gewicht/Volumen) Vitamin E, 0,001 bis 1,0 Prozent (Gewicht/Volumen) Vitamin A, 0,001 bis 1,0 Prozent (Gewicht/Volumen) Vitamin D und 0,001 bis 1,0 Prozent (Gewicht/Volumen) Vitamin C.

13. Zusammensetzung nach Anspruch 5, wobei die Zusammensetzung ferner eine oder mehrere Aminosäuren und/oder physiologisch annehmbare Salze umfasst, die aus einer Gruppe ausgewählt sind, umfassend Glutaminsäure, Glutamin, Asparaginsäure, Asparagin, Isoleucin, Phenylalanin, Valin, Leucin, Prolin und Threonin besteht, wobei der Gesamtgehalt an Aminosäuren im Bereich von 0,1 bis 10,0 Prozent (Gewicht/Volumen) liegt.

14. Zusammensetzung nach Anspruch 13, wobei der Gehalt an Aminosäure 1,0 bis 5,0 Prozent (Gewicht/Volumen) beträgt und die Aminosäure Glutaminsäure und/oder Asparaginsäure ist.

15. Verfahren zur Herstellung einer Zusammensetzung für die Vagina, wobei das Verfahren das Hinzufügen der folgenden Mittel zu Wasser und/oder einer wasserlöslichen Gelmatrix und/oder einer emulgierenden Salbenmatrix umfasst: (1) 2-Phenylethanol, dessen Gesamtgehalt 0,05 bis 0,60 Prozent (Gewicht/Volumen) beträgt; (2) Propionsäure und/oder Natriumpropionat und/oder Calciumpropionat, deren Gesamtgehalt, berechnet als Natriumpropionat, 0,25 bis 0,70 Prozent (Gewicht/Volumen) beträgt, und/oder Benzoesäure und/oder Natriumbenzoat, deren Gesamtgehalt, berechnet als Natriumbenzoat, 0,05 bis 0,15 Prozent (Gewicht/Volumen) beträgt; wobei der pH-Wert der Zusammensetzung 3,0 bis 4,3 beträgt und die Darreichungsform der Zusammensetzung eine Lösung, ein wasserlösliches Gel oder eine emulgierende Salbe ist.

## Revendications

1. Composition pour le vagin utilisée pour promouvoir la restauration des lactobacilles bénéfiques, augmenter l'acidité vaginale, éliminer et/ou soulager les inconforts vaginaux comprenant le prurit ou la douleur vulvovaginale, et/ou la douleur des rapports sexuels, améliorer les propriétés de la leucorrhée, éliminer l'odeur de la leucorrhée, et/ou prévenir et/ou traiter les maladies infectieuses vaginales comprenant la vaginose cytolytique, la dysbactériose vaginale, la vaginite atrophique, la vaginite aérobie, et/ou la vaginose bactérienne, dans laquelle la composition comprend les agents antimicrobiens suivants : (1) 2-phényléthanol, dont la teneur totale est de 0,05 à 0,60 pour cent (poids/volume) ; (2) acide propionique et/ou propionate de sodium et/ou propionate de calcium, dont la teneur totale, calculée en propionate de sodium, est de 0,25 à 0,70 pour cent (poids/volume), et/ou de l'acide benzoïque et/ou du benzoate de sodium, dont la teneur totale, calculée en benzoate de sodium, est de 0,05 à 0,15 pour cent (poids/volume) ; dans laquelle la valeur pH de la composition est compris entre 3,0 à 4,3, et la forme posologique de la composition est une solution, un gel hydrosoluble ou une pommade émulsive.

2. La composition pour le vagin selon la revendication 1 utilisée pour promouvoir la restauration des lactobacilles bénéfiques, augmenter l'acidité vaginale, éliminer et/ou soulager les inconforts vaginaux comprenant le prurit ou la douleur vulvovaginale, et/ou la douleur des rapports sexuels, améliorer les propriétés de la leucorrhée, éliminer l'odeur de la leucorrhée, et/ou prévenir et/ou traiter les maladies infectieuses vaginales comprenant la vaginose cytolytique, la dysbactériose vaginale, la vaginite atrophique, la vaginite aérobie, et/ou la vaginose bactérienne, dans laquelle la teneur totale en 2-phényléthanol est comprise entre 0,10 à 0,40 pour cent (poids/volume) ; la teneur totale en acide propionique et/ou en propionate de sodium et/ou en propionate de calcium, calculée en propionate de sodium, est comprise entre 0,525 à 0,575 pour cent (poids/volume) ; la teneur totale en acide benzoïque et/ou en benzoate de sodium, calculée en benzoate de sodium, est comprise entre 0,065 à 0,090 pour cent (poids/volume).

3. La composition pour le vagin selon la revendication 1 utilisée pour promouvoir la restauration des lactobacilles bénéfiques, augmenter l'acidité vaginale, éliminer et/ou soulager les inconforts vaginaux comprenant le prurit ou la douleur vulvovaginale, et/ou la douleur des rapports sexuels, améliorer les propriétés de la leucorrhée, éliminer l'odeur de la leucorrhée, et/ou prévenir et/ou traiter les maladies infectieuses vaginales comprenant la vaginose cytolytique, la dysbactériose vaginale, la vaginite atrophique, la vaginite aérobie, et/ou la vaginose bactérienne, dans laquelle les agents antimicrobiens étant le 2-phényléthanol, l'acide propionique et/ou le propionate de sodium, et l'acide benzoïque et/ou le benzoate de sodium.

4. La composition pour le vagin selon la revendication 1 utilisée pour promouvoir la restauration des lactobacilles bénéfiques, augmenter l'acidité vaginale, éliminer et/ou soulager les inconforts vaginaux comprenant le prurit ou la douleur vulvovaginale, et/ou la douleur des rapports sexuels, améliorer les propriétés de la leucorrhée, éliminer l'odeur de la leucorrhée, et/ou prévenir et/ou traiter les maladies infectieuses vaginales comprenant la vaginose cytolytique, la dysbactériose vaginale, la vaginite atrophique, la vaginite aérobie, et/ou la vaginose bactérienne, dans laquelle la composition pour le vagin comprend en outre un ou plusieurs agents antimicrobiens choisis dans un groupe constitué de acide déhydroacétique, déshydroacétate de sodium, acide sorbique, sorbate de potassium, sorbate de sodium, acide diacétique, diacétate de sodium, acide caprylique, caprylate de sodium, acide caprique, caprate de sodium, acide undécylénique, undécylénate de sodium, acide laurique, laurate de sodium, natamycine, lactoferrine, peptide de lactoferrine, lysozyme, protéine antibactérienne, peptide antibactérien, acide lichénique, bergénine, tropolone, acide chlorogénique, palmatine, alcool benzylique, propylène phénoxyéthanol, 1,2-pentanediol, 1,2-hexanediol, 1,6-hexanediol, 1,2-octanediol, 1,2-décanediol, 2-méthyl-1,3-propanediol, éthylhexylglycérine et peroxyde de benzoyle.

5. Composition pour le vagin, dans laquelle la composition comprend : (1) 2-phényléthanol, dont la teneur totale est de 0,05 à 0,60 pour cent (poids/volume) ; (2) acide propionique et/ou du propionate de sodium et/ou du propionate de calcium, dont la teneur totale, calculée en propionate de sodium, est de 0,25 à 0,70 pour cent (poids/volume), et/ou acide benzoïque et/ou benzoate de sodium, dont la teneur totale, calculée en benzoate de sodium, est de 0,05 à 0,15 pour cent (poids/volume) ;
dans laquelle la forme posologique de la composition est une solution, un gel hydrosoluble ou une pommade émulsive, dans laquelle la valeur pH de la composition est comprise entre 3,0 à 4,3.

6. Composition selon la revendication 5, dans laquelle la teneur totale en 2-phényléthanol est comprise entre 0,10 à 0,40 pour cent (poids/volume) ; la teneur totale en acide propionique et/ou en propionate de sodium et/ou en propionate de calcium, calculée en tant que propionate de sodium, est comprise entre 0,525 à 0,575 pour cent (poids/volume) ; la teneur totale en acide benzoïque et/ou en benzoate de sodium, calculée en tant que benzoate de sodium, est comprise entre 0,065 à 0,090 pour cent (poids/volume).

7. Composition selon la revendication 5, dans laquelle les agents antimicrobiens sont 2-phényléthanol, acide propionique et/ou propionate de sodium, et acide benzoïque et/ou benzoate de sodium.

8. Composition selon la revendication 5, dans laquelle la composition comprend en outre un ou plusieurs agents antimicrobiens choisis dans un groupe constitué de acide déhydroacétique, déhydroacétate de sodium, acide sorbique, sorbate de potassium, sorbate de sodium, acide diacétique, diacétate de sodium, acide caprylique, caprylate de sodium, acide caprique, caprate de sodium, acide undécylénique, undécylénate de sodium, acide laurique, laurate de sodium, natamycine, lactoferrine, peptide de lactoferrine, lysozyme, protéine antibactérienne, peptide antibactérien, acide lichénique, bergénine, tropolone, acide chlorogénique, palmatine, alcool benzylique, propylène phénoxyéthanol, 1,2-pentanediol, 1,2-hexanediol, 1,6-hexanediol, 1,2-octanediol, 1,2-décanediol, 2-méthyl-1,3-propanediol, éthylhexylglycérine et peroxyde de benzoyle.

9. Composition selon la revendication 5, dans laquelle la composition comprend en outre un ou plusieurs œstrogènes et/ou agents phyto-œstrogènes choisis dans un groupe constitué de diéthylstilbestrol, estradiol, estriol, daidzéine, aglycone de daidzéine, génistine, glycitéine, aglycone de glycitéine, biochanine, coumestrol et formononétine, la teneur totale en œstrogènes et/ou agents phyto-œstrogènes étant comprise entre 0,001 à 1,0 pour cent (poids/volume).

10. Composition selon la revendication 5, dans laquelle la composition comprend en outre un ou plusieurs saccharides choisis dans un groupe constitué de glucose, fructose, mannose, saccharose, isomaltulose, 1-kestose, nistose trihydraté, 1F-fructofuranosylnystose, maltose, isomaltose, isomaltotriose, isomaltotétraose, isomaltopentaose, tréhalose, cellobiose, melibiose, gentiobiose, gentiooligosaccharide, raffinose, panose, maltooligosaccharide, isomaltulooligosaccharide, fructooligosaccharide, glucomannane, dextrine, amidon et glycogène, la teneur totale en saccharides étant comprise entre 1,0 à 9,0 pour cent (poids/volume).

11. Composition selon la revendication 10, dans laquelle les saccharides sont choisis dans un groupe constitué de glucose, fructose, mannose, saccharose, maltose, isomaltulose, isomaltose, tréhalose et maltooligosaccharide, avec une teneur totale de 2,0 à 6,5 pour cent (poids/volume).

12. Composition selon la revendication 5, dans laquelle la composition comprend en outre une ou plusieurs substances choisies dans un groupe constitué de 0,01 à 5,0 pour cent (poids/volume) d'extrait d'aloès, 0,01 à 5,0 pour cent (poids/volume) d'extrait de lavande, 0,001 à 1,0 pour cent (poids/volume) de vitamine E, 0,001 à 1,0 pour cent (poids/volume) de vitamine A, 0,001 à 1,0 pour cent (poids/volume) de vitamine D et 0,001 à 1,0 pour cent (poids/volume) de vitamine C.

13. Composition selon la revendication 5, dans laquelle la composition comprend en outre un ou plusieurs acides aminés et/ou des sels physiologiquement acceptables, choisis dans un groupe constitué d'acide glutamique, glutamine, acide aspartique, asparagine, isoleucine, phénylalanine, valine, leucine, proline et thréonine, la teneur totale en acides aminés étant comprise entre 0,1 à 10,0 pour cent (poids/volume).

14. Composition selon la revendication 13, dans laquelle la teneur en acide aminé est de 1,0 à 5,0 pour cent (poids/volume), et l'acide aminé est acide glutamique et/ou acide aspartique.

15. Méthode de préparation d'une composition pour le vagin, dans laquelle la méthode comprend l'opération d'ajout des agents suivants dans l'eau, et/ou la matrice de gel soluble dans l'eau, et/ou la matrice de pommade émulsive : (1) 2-phényléthanol, dont la teneur totale est de 0,05 à 0,60 pour cent (poids/volume) ; (2) acide propionique et/ou propionate de sodium et/ou propionate de calcium, dont la teneur totale, calculée en tant que propionate de sodium, est de 0,25 à 0,70 pour cent (poids/volume), et/ou acide benzoïque et/ou benzoate de sodium, dont la teneur totale, calculée en benzoate de sodium, est de 0,05 à 0,15 pour cent (poids/volume) ; dans laquelle la valeur pH de la composition est compris entre 3,0 à 4,3, et la forme posologique de la composition est une solution, un gel hydrosoluble ou une pommade émulsive.
